(19) Europäisches Patentamt
European Patent Office
Office européen des brevets

(11) EP 4 353 729 A1

(12) **EUROPEAN PATENT APPLICATION**
published in accordance with Art. 153(4) EPC

(43) Date of publication:
**17.04.2024 Bulletin 2024/16**

(21) Application number: **22806833.4**

(22) Date of filing: **12.05.2022**

(51) International Patent Classification (IPC):
***C07D 498/04*** *(2006.01)* ***A61K 31/4188*** *(2006.01)*
***A61P 35/00*** *(2006.01)*

(52) Cooperative Patent Classification (CPC):
**A61K 31/4188; A61P 35/00; C07D 498/04**

(86) International application number:
**PCT/CN2022/092465**

(87) International publication number:
**WO 2022/237871 (17.11.2022 Gazette 2022/46)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **13.05.2021 PCT/CN2021/093552**
**09.05.2022 CN 202210506024**

(71) Applicant: **Betta Pharmaceuticals Co., Ltd**
**Yuhang**
**Hangzhou**
**Zhejiang 311100 (CN)**

(72) Inventors:
- **ZU, Houxian**
  **Beijing 100176 (CN)**
- **CHEN, Liang**
  **Hangzhou, Zhejiang 311100 (CN)**
- **SONG, Xizhen**
  **Beijing 100176 (CN)**
- **ZHAO, Xintao**
  **Hangzhou, Zhejiang 311100 (CN)**
- **CHEN, Kai**
  **Beijing 100176 (CN)**
- **LIU, Xiaoyun**
  **Beijing 100176 (CN)**
- **WANG, Jin**
  **Beijing 100176 (CN)**
- **XIA, Zhifang**
  **Hangzhou, Zhejiang 311100 (CN)**
- **XU, Ying**
  **Beijing 100176 (CN)**
- **ZHOU, Yuanzheng**
  **Beijing 100176 (CN)**
- **DING, Lieming**
  **Hangzhou, Zhejiang 311100 (CN)**
- **WANG, Jiabing**
  **Beijing 100176 (CN)**

(74) Representative: **Graf von Stosch**
**Patentanwaltsgesellschaft mbH**
**Prinzregentenstraße 22**
**80538 München (DE)**

(54) **POLYMORPH OF IMIDAZOLIDINONE COMPOUND, PREPARATION METHOD THEREFOR AND USE THEREOF**

(57) Disclosed are a polymorph of (*S*)-1-(2-((*S*)-3-cyclopropyl-5-isopropyl-2,4-dioxoimidazolidin-1-yl)-5,6-dihydrobenzo[*f*]imidazo[1,2-*d*][1,4]oxazepin-9-yl)pyrrolidine-2-carboxamide, a composition comprising the polymorph, a use method therefor and a preparation method therefor.

EP 4 353 729 A1

## Description

## TECHNICAL FIELD

The present invention relates to a polymorph of (*S*)-1-(2-((*S*)-3-cyclopropyl-5-isopropyl-2,4-dioxoimidazolidin-1-yl)-5,6-dihydrobenzo[*f*] imidazo[1,2-*d*][1,4]oxazepin-9-yl)pyrrolidine-2-carboxamide. The present invention also relates to a method for preparing the polymorph, a pharmaceutical composition containing the polymorph, and use of the polymorph of the compound and the pharmaceutical composition thereof in the treatment of a PI3K-mediated disease.

## BACKGROUND

Phosphatidylinositol 3-kinase (PI3K) signaling pathway is a key signaling pathway that controls life activities such as growth, proliferation, survival, differentiation, metastasis, and apoptosis in cells. Since PI3K, Akt and mTOR are key sites in this pathway, the signaling pathway is called PI3K/Akt/mTOR signaling pathway. In recent years, PI3K inhibitors have become a research hotspot for antitumor medicament all over the world.

PI3K activated by RTK or Ras catalyzes the generation of phosphatidylinositol-3,4,5-trisphosphate (PIP3) from phosphatidylinositol-3,4-bisphosphate (PIP2). PIP3 binds to protein kinases such as Akt and phosphatidylinositol 3-phosphate (PIP)-dependent protein kinase (PDK), which activates Akt by phosphorylating it and transfers Akt from the cytoplasm into the nucleus. Activated Akt can further phosphorylate downstream effector substrates to affect cellular activities such as cell survival, cell cycle, growth, and the like (Ma K, Cheung SM, Marshall AJ et al., Cell Signal, 2008, 20:684-694). Therefore, the activation of the PI3K/Akt/mTOR signaling pathway may inhibit apoptosis, enhance cellular tolerance, promote cell survival and proliferation, and participate in angiogenesis, thus promoting tumor growth and metastasis.

Phosphatidylinositol 3-kinase (PI3K) belongs to the Lipid kinase family, and members of this family are classified into three types: type I, type II and type III, depending on the mechanism of PI3K activation and structural characteristics (Vanhaesebroeck B, Waterfield MD; Exp Cell Res, 1999, 253:239-254). Currently, the relatively thoroughly studied type is PI3K of type I. Depending on the type of cell surface receptor, PI3K of type I is further divided into two distinct subtypes, IA and IB, which receive delivered signals from tyrosine protein kinase receptors (RTKs) and G protein-coupled receptors (GPCRs), respectively (Wu P, Liu T, Hu Y; Curr Med Chem, 2009, 16:916-930). PI3K of type IA includes three subtypes: PI3K$\alpha$, PI3K$\beta$ and PI3K$\delta$, and PI3K of type IB includes only one subtype: PI3K$\gamma$. PI3K of Type II is divided into three subtypes: PI3KC2$\alpha$, PI3KC2$\beta$, and PI3KC2$\gamma$, depending on C-terminal structure, but their *in vivo* substrates are not yet clear, and the understanding of their mechanism of action and specific functions is relatively limited (Falasca M, T. Muffucci; Biochem Soc Trans, 2007, 35:211-214). PI3K of Type III has only one member, Vps34 (Vacuolar Protein Sorting 34), which acts as a calibrator at the protein level in regulating the downstream mTOR signaling cascade (Schu P, Takegawa. K, Fry. M et al., Science, 1993, 260:88-91).

Among the four subtypes of PI3K of type I, PI3K$\alpha$ and PI3K$\beta$ are expressed in a variety of organs, whereas PI3K$\delta$ and PI3K$\gamma$ are predominantly distributed in myeloid cells (Kong D, Yamori T; Cancer Sci, 2008, 99:1734-1740). Among them, PI3K$\alpha$ has the closest association with tumor development. The gene encoding the catalytic subunit p110$\alpha$ of PI3K$\alpha$ is PIK3CA, and its mutations are prevalent in a variety of malignant tumors, including breast cancer, colon cancer, endometrial carcinoma, gastric cancer, ovarian cancer, and lung cancer (Steelman. LS, Chappell. WH, Abrams. SL et al., Aging, 2011, 3:192-222). Aberrant activation of PI3K$\alpha$ may up-regulate the activated PI3K signaling pathway and promote excessive cell proliferation, growth and metastasis, leading to the formation of tumor. While the other three subtypes, PI3K$\beta$, PI3K$\delta$, and PI3K$\gamma$, play roles in thrombosis, immune function, and allergic and inflammatory responses, respectively, they also play important roles in tumorigenesis by affecting catalytic activity, physicochemical properties, interactions, and recognition.

Early relatively thoroughly studied PI3K inhibitors are wortmannin and LY294002, known as the first generation of PI3K inhibitors, both of which play an important role in the study of the physiological function of PI3K and the mechanism of the signaling pathway, and the like, providing an important foundation for subsequent studies. Through the study of wortmannin and LY294002, the second generation of PI3K inhibitors with newer structures, higher activity and better pharmacokinetic properties have been developed, including morpholino aryl compounds, imidazolopyridines and imidazolylquinolines, and the like, which have brought new hope to tumor therapy. Dozens of PI3K inhibitors have been in the clinical research stage, which are mainly divided into pan-PI3K inhibitors, dual PI3K/mTOR inhibitors and PI3K subtype-specific inhibitors.

Pictilisib (GDC-0941 or RG7321) is a potent multi-target (pan) PI3K of type I isotype inhibitor with moderate selectivity for p110$\beta$ and p110$\gamma$, and can competitively bind to the ATP-binding pocket of PI3K to block the formation of phosphatidylinositol 3,4,5-trisphosphate (PIP 3), the second messenger transmitting downstream signaling of PI3K.

Taselisib (GDC-0032 or RG7604) is an effective PI3K inhibitor with potent PI3K activity developed by Genentech (WO2011036280), a $\beta$-isotype sparing-inhibitor of the catalytic subunit of PI3K, with 31-fold greater selectivity for the $\alpha$-

subunit compared to the β-subunit.

Alpelisib (BYL719) is the first PI3Kα-selective inhibitor developed by Novartis with 5 nM inhibitory activity against p110α. Preclinical data shows that BYL719 can inhibit the phosphorylation of Akt, blocking the PI3K signaling pathway and inhibiting the growth of breast cancer cells containing the PIK3CA mutation (Dejan Juric et al., Cancer Res, 2012, 72:1). This compound has been approved for marketing by the U.S. Food and Drug Administration (FDA) in 2019, in combination with fulvestrant, for the treatment of postmenopausal female patients and male patients with PIK3CA mutations, HR+/HER2-advanced or metastatic breast cancer and disease progression during or after endocrinotherapy. Meanwhile the preliminary results show that this PI3Kα-specific small molecule inhibitor is promising in the treatment of diseases such as head and neck cancer, ovarian cancer, triple-negative breast cancer, HER2+ breast cancer, and PIK3CA-associated overgrowth disease spectrum, and the like.

Benzoxazepin oxazolidinone compounds with PI3K modulating activity or function are disclosed in WO2017001658, and it is disclosed in the specification that when compared to Taselisib and Alpelisib, representative compounds of the invention exhibit strong activity against PI3Kα and exhibit a significantly enhanced selectivity against other isotypes of PI3Kβ, PI3Kδ and PI3Kγ.

In order to achieve better tumor therapeutic efficacy, to better meet the market demand, and to provide a new medicament option in clinic, we hope to develop a new generation of PI3Kα inhibitors with higher activity, better pharmacokinetics, and/or lower toxicity.

## SUMMARY

The present invention relates to a polymorph of compound (*S*)-1-(2-((*S*)-3-cyclopropyl-5-isopropyl-2,4-dioxoimidazolidin-1-yl)-5,6-dihydrobenzo[*f*]imidazo[1,2-*d*][1,4]oxazepin-9-yl)pyrrolidine-2-carboxamide (hereinafter referred to as "Compound 1"), and Compound 1 is represented by the following structure:

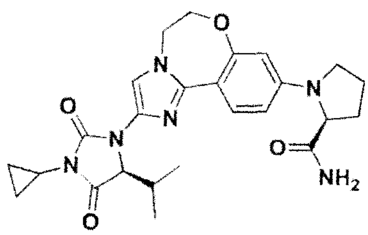

.

Further, the polymorph of Compound 1 is an anhydrous crystal form, a hydrate and/or a solvate of Compound 1.

The present invention also relates to a pharmaceutical composition comprising a polymorph of Compound 1 and a pharmaceutically acceptable excipient, adjuvant and/or carrier.

The present invention also relates to use of the polymorph of Compound 1 and the pharmaceutical composition in the treatment of PI3K-mediated diseases.

The present invention also relates to a method for treating cancer, comprising administering to a subject in need thereof a therapeutically effective amount of the polymorph of Compound 1 or the pharmaceutical composition containing the same.

The present invention also includes methods of preparing Compound 1 and the polymorph thereof.

## BRIEF DESCRIPTION OF THE DRAWINGS

FIG. 1: X-ray powder diffraction pattern of crystal form A of Compound 1.
FIG. 2: Thermogravimetric analysis (TGA) pattern of crystal form A of Compound 1.
FIG. 3: Differential scanning calorimetry (DSC) pattern of crystal form A of Compound 1.
FIG. 4: X-ray powder diffraction pattern of crystal form B-2 of Compound 1.
FIG. 5: X-ray powder diffraction pattern of crystal form B-3 of Compound 1.
FIG. 6: X-ray powder diffraction pattern of crystal form D-1 of Compound 1.
FIG. 7: X-ray powder diffraction pattern of crystal form D-2 of Compound 1.
FIG. 8: X-ray powder diffraction pattern of crystal form D-3 of Compound 1.
FIG. 9: X-ray powder diffraction pattern of crystal form E-1 of Compound 1.
FIG. 10: X-ray powder diffraction pattern of crystal form E-2 of Compound 1.
FIG. 11: X-ray powder diffraction pattern of crystal form E-3 of Compound 1.

FIG. 12: X-ray powder diffraction pattern of crystal form F of Compound 1.
FIG. 13: X-ray powder diffraction pattern of crystal form G of Compound 1.
FIG. 14: X-ray powder diffraction pattern of crystal form H of Compound 1.
FIG. 15: X-ray powder diffraction pattern of crystal form I of Compound 1.
FIG. 16: X-ray powder diffraction pattern of crystal form J of Compound 1.
FIG. 17: X-ray powder diffraction pattern of crystal form K of Compound 1.
FIG. 18: X-ray powder diffraction pattern of crystal form B-1 of Compound 1.
FIG. 19: TGA pattern of crystal form B-1 of Compound 1.
FIG. 20: DSC pattern of crystal form B-1 of Compound 1.
FIG. 21: X-ray powder diffraction pattern of crystal form C of Compound 1.
FIG. 22: TGA pattern of crystal form C of Compound 1.
FIG. 23: DSC pattern of crystal form C of Compound 1.
FIG. 24: XRPD overlap pattern of crystal form D-1 of Compound 1 before and after heating.
FIG. 25: XRPD overlap pattern of crystal form E-1 of Compound 1 before and after heating.
FIG. 26: XRPD overlap pattern of crystal form F of Compound 1 before and after heating.
FIG. 27: XRPD overlap pattern of crystal form G of Compound 1 before and after heating.
FIG. 28: XRPD overlap pattern of crystal form H of Compound 1 before and after heating.
FIG. 29: XRPD overlap pattern of crystal form I of Compound 1 before and after heating.
FIG. 30: XRPD overlap pattern of crystal form J of Compound 1 before and after heating.
FIG. 31: Results of grinding sensitivity assay for crystal form B-1 of Compound 1.
FIG. 32: Results of grinding sensitivity assay for crystal form C of Compound 1.
FIG. 33: XRPD pattern of crystal form M of Compound 1.

## DETAILED DESCRIPTION

The present invention relates to a polymorph of compound (*S*)-1-(2-((*S*)-3-cyclopropyl-5-isopropyl-2,4-dioxoimidazolidin-1-yl)-5,6-dihydrobenzo[*f*]imidazo[1,2-*d*][1,4]oxazepin-9-yl)pyrrolidine-2-carboxamide (hereinafter referred to as "Compound 1"), and Compound 1 is represented by the following structure:

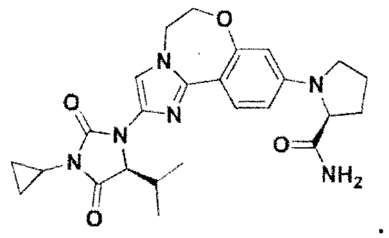

As mentioned above, Compound 1 is present in the polymorph of the invention as a free base, i.e., in a non-salt form. Therefore, the term "Compound 1" refers to (*S*)-1-(2-((*S*)-3-cyclopropyl-5-isopropyl-2,4-dioxoimidazolidin-1-yl)-5,6-dihydrobenzo[*f*]imidazo[1,2-d][1,4]oxazepin-9-yl)pyrrolidine-2-carboxamide (free base).

Compound 1 provided in the present invention has been found to form crystalline solvates with a variety of solvents. Specifically, the present invention relates to dichloromethane, acetone, n-propanol, 2-butanone, isopropanol, dimethyl sulfoxide, *N,N*-dimethylformamide, tetrahydrofuran, acetonitrile, 1,4-dioxane and trichloromethane solvates of Compound 1.

In some embodiments, the polymorph of Compound 1 is crystal form A with X-ray powder diffraction pattern having characteristic peaks at 20 of 6.2°±0.2°, 7.3°±0.2°, 9.1°±0.2°, 13.6°±0.2° and 14.2°±0.2°.

As a preferred embodiment, the X-ray powder diffraction pattern of the crystal form A has characteristic peaks at 2θ of 6.2°±0.2°, 7.3°±0.2°, 9.1°±0.2°, 13.6°±0.2°, 14.2°±0.2°, 14.9°±0.2°, 17.1°±0.2° and 17.3°±0.2°.

As a preferred embodiment, the X-ray powder diffraction pattern of the crystal form A has characteristic peaks at 2θ of 6.2°±0.2°, 7.3°±0.2°, 9.1°±0.2°, 11.5°±0.2°, 13.6°±0.2°, 14.2°±0.2°, 14.9°±0.2°, 17.1°±0.2°, 17.3°±0.2° and 18.1°±0.2°.

In some embodiments, the crystal form A is a dichloromethane solvate of Compound 1.

In some embodiments, the X-ray powder diffraction pattern of crystal form A has main characteristic peaks shown in the table below:

| Number | 2θ±0.2(°) | d Value [Å] | Relative Intensity (%) |
|---|---|---|---|
| 1 | 6.228 | 14.180 | 100 |
| 2 | 7.291 | 12.115 | 11.5 |
| 3 | 9.095 | 9.716 | 63.4 |
| 4 | 11.486 | 7.698 | 2.4 |
| 5 | 12.207 | 7.244 | 0.4 |
| 6 | 12.540 | 7.053 | 0.4 |
| 7 | 13.240 | 6.682 | 0.9 |
| 8 | 13.590 | 6.510 | 2.8 |
| 9 | 14.192 | 6.235 | 7.9 |
| 10 | 14.660 | 6.037 | 2.9 |
| 11 | 14.911 | 5.937 | 0.8 |
| 12 | 16.542 | 5.355 | 0.2 |
| 13 | 17.058 | 5.194 | 12.8 |
| 14 | 17.257 | 5.135 | 12.4 |
| 15 | 18.083 | 4.902 | 1.7 |
| 16 | 18.279 | 4.849 | 1.0 |
| 17 | 18.517 | 4.788 | 0.7 |
| 18 | 19.679 | 4.508 | 1.2 |
| 19 | 20.058 | 4.423 | 1.1 |
| 20 | 20.619 | 4.304 | 4.1 |
| 21 | 20.803 | 4.267 | 1.5 |
| 22 | 22.095 | 4.020 | 4.3 |
| 23 | 23.075 | 3.851 | 7.8 |
| 24 | 23.752 | 3.743 | 1.3 |
| 25 | 24.597 | 3.616 | 0.5 |
| 26 | 25.520 | 3.488 | 1.1 |
| 27 | 26.000 | 3.424 | 2.7 |
| 28 | 27.613 | 3.228 | 0.7 |
| 29 | 27.989 | 3.185 | 0.7 |
| 30 | 28.662 | 3.112 | 1.2 |
| 31 | 30.145 | 2.962 | 0.6 |
| 32 | 30.972 | 2.885 | 0.9 |
| 33 | 31.678 | 2.822 | 0.9 |
| 34 | 33.827 | 2.648 | 0.4 |
| 35 | 34.690 | 2.584 | 0.6 |
| 36 | 35.731 | 2.511 | 0.8 |
| 37 | 38.978 | 2.309 | 1.0 |

In some embodiments, the crystal form A has an X-ray powder diffraction pattern substantially as shown in FIG. 1.

In some embodiments, the crystal form A further has a thermogravimetric analysis (TGA) pattern substantially as shown in FIG. 2 and/or a differential scanning calorimetry (DSC) pattern substantially as shown in FIG. 3.

In some embodiments, acetonitrile solvate I of Compound I (crystal form B-2) shows characteristic peaks in the X-ray powder diffraction pattern at 2θ of about 6.1°±0.2°, 7.7°±0.2°, 9.3°±0.2°, 14.1°±0.2°, 15.4°±0.2°, 16.9°±0.2° and 17.3°±0.2°. In some embodiments, the crystal form B-2 is characterized by an X-ray powder diffraction pattern substantially as shown in FIG. 4.

In some embodiments, dichloromethane solvate II of Compound I (crystal form B-3) shows characteristic peaks in the X-ray powder diffraction pattern at 20 of about 6.1°±0.2°, 7.7°±0.2°, 9.3°±0.2°, 14.1°±0.2°, 15.4°±0.2°, 16.9°±0.2° and 17.2°±0.2°. Further, the crystal form B-3 is characterized by an X-ray powder diffraction pattern substantially as shown in FIG. 5.

In some embodiments, acetone solvate of Compound I (crystal form D-1) shows characteristic peaks in the X-ray powder diffraction pattern at 2θ of about 6.1°±0.2°, 6.5°±0.2°, 6.8°±0.2°, 7.6°±0.2°, 8.9°±0.2° and 9.3°±0.2°. Further, the crystal form D-1 is characterized by an X-ray powder diffraction pattern substantially as shown in FIG. 6.

In some embodiments, n-propanol solvate of Compound I (crystal form D-2) shows characteristic peaks in the X-ray powder diffraction pattern at 2θ of about 6.1°±0.2°, 6.4°±0.2°, 6.9°±0.2°, 7.6°±0.2°, 8.9°±0.2° and 9.3°±0.2°. Further, the crystal form D-2 is characterized by an X-ray powder diffraction pattern substantially as shown in FIG. 7.

In some embodiments, 2-butanone solvate of Compound I (crystal form D-3) shows characteristic peaks in the X-ray powder diffraction pattern at 2θ of about 6.1°±0.2°, 6.6°±0.2°, 7.6°±0.2°, 8.8°±0.2°, 9.3°±0.2°, 17.2°±0.2°, 24.2°±0.2° and 25.2°±0.2°. Further, the crystal form D-3 is characterized by an X-ray powder diffraction pattern substantially as shown in FIG. 8.

In some embodiments, 1,4-dioxane solvate of Compound I (crystal form E-1) shows characteristic peaks in the X-ray powder diffraction pattern at 20 of about 6.2°±0.2°, 6.9°±0.2°, 8.8°±0.2°, 9.2°±0.2°, 16.9°±0.2°, 17.4°±0.2° and 17.7°±0.2°. Further, the crystal form E-1 is characterized by an X-ray powder diffraction pattern substantially as shown in FIG. 9.

In some embodiments, tetrahydrofuran solvate I of Compound I (crystal form E-2) shows characteristic peaks in the X-ray powder diffraction pattern at 2θ of about 6.3° ± 0.2°, 7.0° ± 0.2°, 7.6° ± 0.2°, 8.8° ± 0.2°, 9.3° ± 0.2°, 17.0° ± 0.2°, 17.5° ± 0.2° and 17.7° ± 0.2°. Further, the crystal form E-2 is characterized by an X-ray powder diffraction pattern substantially as shown in FIG. 10.

In some embodiments, isopropanol solvate I of Compound I (crystal form E-3) shows characteristic peaks in the X-ray powder diffraction pattern at 2θ of about 6.3°±0.2°, 6.9°±0.2°, 7.6°±0.2°, 8.9°±0.2°, 9.2°±0.2°, 17.6°±0.2° and 21.0°±0.2°. Further, the crystal form E-3 is characterized by an X-ray powder diffraction pattern substantially as shown in FIG. 11.

In some embodiments, dimethyl sulfoxide solvate of Compound I (crystal form F) shows characteristic peaks in the X-ray powder diffraction pattern at 20 of about 6.4°±0.2°, 6.8°±0.2°, 8.8°±0.2°, 12.5°±0.2°, 13.7°±0.2° and 20.7°±0.2°. Further, the crystal form F is characterized by an X-ray powder diffraction pattern substantially as shown in FIG. 12.

In some embodiments, N,N-dimethylformamide solvate of Compound I (crystal form G) shows characteristic peaks in the X-ray powder diffraction pattern at 2θ of about 6.2°±0.2°, 7.0°±0.2°, 8.9°±0.2°, 14.0°±0.2° and 21.2°±0.2°. Further, the crystal form G is characterized by an X-ray powder diffraction pattern substantially as shown in FIG. 13.

In some embodiments, tetrahydrofuran solvate II of Compound I (crystal form H) shows characteristic peaks in the X-ray powder diffraction pattern at 2θ of about 6.3°±0.2°, 7.0°±0.2°, 8.9°±0.2°, 12.5°±0.2°, 13.6°±0.2°, 14.0°±0.2°, 15.0°±0.2°, 17.0°±0.2°, 17.5°±0.2° and 18.6°±0.2°. Further, the crystal form H is characterized by an X-ray powder diffraction pattern substantially as shown in FIG. 14.

In some embodiments, acetonitrile solvate II of Compound I (crystal form I) shows characteristic peaks in the X-ray powder diffraction pattern at 20 of about 6.1°±0.2°, 6.5°±0.2°, 6.8°±0.2°, 7.1°±0.2°, 7.6°±0.2°, 8.8°±0.2°, 9.1°±0.2° and 9.3°±0.2°. Further, the crystal form I is characterized by an X-ray powder diffraction pattern substantially as shown in FIG. 15.

In some embodiments, isopropanol solvate II of Compound I (crystal form J) shows characteristic peaks in the X-ray powder diffraction pattern at 20 of about 6.4°±0.2°, 6.7°±0.2°, 8.7±0.2°, 8.8°±0.2°, 11.6°±0.2°, 13.0°±0.2°, 13.4°±0.2° and 21.0°±0.2°. Further, the crystal form J is characterized by an X-ray powder diffraction pattern substantially as shown in FIG. 16.

In some embodiments, trichloromethane solvate of Compound I (crystal form K) shows characteristic peaks in the X-ray powder diffraction pattern at 2θ of about 6.5°±0.2°, 8.9°±0.2°, 13.1±0.2°, 17.5°±0.2° and 17.8°±0.2°. Further, the crystal form K is characterized by an X-ray powder diffraction pattern substantially as shown in FIG. 17.

Due to the use of the compound in the pharmaceutical field, the crystal form of Compound 1 is preferably a pharmaceutically acceptable crystal form. These pharmaceutically acceptable crystal forms include solvate and non-solvate forms. The International Council For Harmonisation Of Technical Requirements for Registration of Pharmaceuticals for Human use, ICH Harmonised Guideline, Impurities: GUIDELINE FOR RESIDUAL SOLVENTS Residual Solvents Q3C(R8), recommends the specific amounts of residual solvents that are considered safe for use in pharmaceuticals. The term "permitted daily exposure" (PDE) is defined as a pharmaceutically acceptable intake of residual solvents. Residual solvents are evaluated for their possible risks to human health and are classified into three types as follows:

Class I solvents: solvents to be avoided, which are known human carcinogens, solvents with unacceptable toxicity or environmental hazards, or strongly suspected human carcinogens.
Class II solvents: solvents to be limited with certain toxicities, which are non-genotoxic animal carcinogens or solvents possible causative of other irreversible toxicity such as neurotoxicity or teratogenicity, or solvents suspected of other significant but reversible toxicities.
Class III solvents: low toxic solvents which have low toxic potential and little harm to human, and no limit to health-based exposure is needed. PDEs of Class III solvents are about 50 mg per day.

Solvents typically present in pharmaceutically acceptable solvates include those of Class III. As disclosed herein, acetone, n-propanol, isopropanol and dimethyl sulfoxide are Class III solvents in the solvates. Although Class III solvents are pharmaceutically acceptable, their amount must be controlled within the PDE. However, their potential adverse effects still cannot be eliminated, which is thus unfavorable for medicament development.

It was found that the crystals of Compound 1 of the present invention have special properties, most of the common organic solvents can enter the crystal lattice during the crystallization of Compound 1, and then form the corresponding solvates, which creates a great obstacle to medicament development. Through a lot of experimental research and efforts, an anhydrous crystal form of Compound 1 was surprisingly developed. Compared to solvates, the anhydrous crystal form of Compound 1 is particularly favorable, substantially eliminates the problem of potential solvent toxicity brought about by solvents and is more suitable for pharmaceutical use. In another aspect, compared to solvates, the anhydrous crystal form B-1 and anhydrous crystal form C provided in the present application are more thermodynamically stable, and more favorable for further processing and preparation of pharmaceutical compositions.

In some embodiments, the present invention provides an anhydrous crystal form B-1 of Compound 1, wherein the X-ray powder diffraction pattern thereof has characteristic peaks at 2θ of 6.1°±0.2°, 7.6°±0.2°, 9.3°±0.2° and 15.4°±0.2°.

As a preferred embodiment, the X-ray powder diffraction pattern of the crystal form B-1 has characteristic peaks at 20 of 6.1°±0.2°, 7.6°±0.2°, 9.3°±0.2°, 11.6°±0.2° and 15.4°±0.2°.

As a preferred embodiment, the X-ray powder diffraction pattern of the crystal form B-1 has characteristic peaks at 2θ of 6.1°±0.2°, 7.6°±0.2°, 9.3°±0.2°, 11.6°±0.2°, 14.1°±0.2° and 15.4°±0.2°.

As a preferred embodiment, the X-ray powder diffraction pattern of the crystal form B-1 has characteristic peaks at 20 of 6.1°±0.2°, 7.6°±0.2°, 9.3°±0.2°, 11.6°±0.2°, 14.1°±0.2°, 15.4°±0.2°, 16.9°±0.2° and 17.2°±0.2°.

As a preferred embodiment, the X-ray powder diffraction pattern of crystal form B-1 has main characteristic peaks shown in the table below:

| Number | 2θ±0.2(°) | d Value [Å] | Relative Intensity (%) |
|---|---|---|---|
| 1 | 4.041 | 21.84726 | 0.4 |
| 2 | 4.242 | 20.81545 | 0.3 |
| 3 | 5.474 | 16.13015 | 0.3 |
| 4 | 6.071 | 14.54681 | 100 |
| 5 | 6.572 | 13.43922 | 0.4 |
| 6 | 7.636 | 11.56898 | 25.4 |
| 7 | 8.404 | 10.51267 | 0.2 |
| 8 | 9.295 | 9.50728 | 82.3 |
| 9 | 11.550 | 7.65551 | 2.8 |
| 10 | 12.216 | 7.23932 | 0.5 |
| 11 | 14.088 | 6.28124 | 4.4 |
| 12 | 14.397 | 6.14745 | 0.6 |
| 13 | 15.372 | 5.75959 | 2.8 |
| 14 | 16.866 | 5.25251 | 7.9 |

(continued)

| Number | 2θ±0.2(°) | d Value [Å] | Relative Intensity (%) |
|---|---|---|---|
| 15 | 17.232 | 5.14167 | 4.8 |
| 16 | 18.717 | 4.73705 | 0.5 |
| 17 | 19.565 | 4.53354 | 1.6 |
| 18 | 20.812 | 4.26462 | 0.5 |
| 19 | 21.460 | 4.13733 | 1.8 |
| 20 | 22.679 | 3.91765 | 1.2 |
| 21 | 23.188 | 3.83283 | 1.8 |
| 22 | 24.197 | 3.67522 | 0.8 |
| 23 | 24.320 | 3.65685 | 0.8 |
| 24 | 25.146 | 3.53867 | 0.4 |
| 25 | 25.810 | 3.44901 | 1.0 |
| 26 | 26.100 | 3.41147 | 0.3 |
| 27 | 26.285 | 3.38783 | 0.4 |
| 28 | 27.519 | 3.23518 | 0.6 |
| 29 | 28.286 | 3.15258 | 0.7 |
| 30 | 28.503 | 3.12902 | 1.2 |
| 31 | 31.773 | 2.81410 | 0.5 |
| 32 | 34.145 | 2.62382 | 0.3 |
| 33 | 34.896 | 2.56903 | 0.8 |
| 34 | 37.135 | 2.41914 | 0.3 |
| 35 | 37.348 | 2.40584 | 0.4 |

As a preferred embodiment, the crystal form B-1 has an X-ray powder diffraction pattern substantially as shown in FIG. 18.

In another aspect, the crystal form B-1 further has a thermogravimetric analysis (TGA) pattern substantially as shown in FIG. 19 and/or a differential scanning calorimetry (DSC) pattern substantially as shown in FIG. 20.

In some embodiments, the present invention provides an anhydrous crystal form C of Compound 1 wherein the X-ray powder diffraction pattern thereof has characteristic peaks at 2θ of 7.0°±0.2°, 8.4°±0.2° and 10.9°±0.2°.

As a preferred embodiment, the crystal form C has characteristic peaks in the X-ray powder diffraction pattern at 20 of 5.5°±0.2°, 7.0°±0.2°, 8.4°±0.2°, 9.8°±0.2°, 10.2°±0.2° and 10.9°±0.2°.

As a preferred embodiment, the crystal form C has characteristic peaks in the X-ray powder diffraction pattern at 20 of 5.5°±0.2°, 7.0°±0.2°, 8.4°±0.2°, 9.8°±0.2°, 10.2°±0.2°, 10.9°±0.2°, 13.5°±0.2° and 13.9°±0.2°.

As a preferred embodiment, the crystal form C has characteristic peaks in the X-ray powder diffraction pattern at 20 of 5.5°±0.2°, 7.0°±0.2°, 8.4°±0.2°, 9.8°±0.2°, 10.2°±0.2°, 10.9°±0.2°, 11.9°±0.2°, 13.5°±0.2°, 13.9°±0.2° and 15.9°±0.2°.

As a preferred embodiment, the crystal form C has characteristic peaks in the X-ray powder diffraction pattern at 20 of 5.5°±0.2°, 7.0°±0.2°, 8.4°±0.2°, 9.8°±0.2°, 10.2°±0.2°, 10.9°±0.2°, 11.9°±0.2°, 13.5°±0.2°, 13.9°±0.2°, 15.9°±0.2° and 17.1°±0.2°.

As a preferred embodiment, the X-ray powder diffraction pattern of crystal form C has main characteristic peaks shown in the table below:

| Number | 2θ±0.2(°) | d Value [Å] | Relative Intensity (%) |
|---|---|---|---|
| 1 | 4.199 | 21.02666 | 1.4 |
| 2 | 5.470 | 16.14392 | 12.8 |

(continued)

| Number | 2θ±0.2(°) | d Value [Å] | Relative Intensity (%) |
|---|---|---|---|
| 3 | 7.023 | 12.57723 | 100.0 |
| 4 | 8.390 | 10.53041 | 40.9 |
| 5 | 9.828 | 8.99269 | 6.9 |
| 6 | 10.195 | 8.66919 | 13.0 |
| 7 | 10.937 | 8.08312 | 35.0 |
| 8 | 11.556 | 7.65120 | 1.2 |
| 9 | 11.872 | 7.44847 | 8.0 |
| 10 | 12.591 | 7.02460 | 0.5 |
| 11 | 13.525 | 6.54154 | 4.3 |
| 12 | 13.940 | 6.34771 | 7.1 |
| 13 | 15.640 | 5.66137 | 0.3 |
| 14 | 15.871 | 5.57943 | 2.7 |
| 15 | 16.415 | 5.39598 | 1.5 |
| 16 | 16.802 | 5.27251 | 3.8 |
| 17 | 17.129 | 5.17255 | 8.1 |
| 18 | 17.495 | 5.06509 | 0.4 |
| 19 | 18.193 | 4.87242 | 0.6 |
| 20 | 18.599 | 4.76695 | 1.2 |
| 21 | 19.724 | 4.49739 | 0.9 |
| 22 | 20.353 | 4.35986 | 0.8 |
| 23 | 20.773 | 4.27263 | 0.4 |
| 24 | 21.972 | 4.04202 | 3.1 |
| 25 | 22.201 | 4.00089 | 4.3 |
| 26 | 23.220 | 3.82752 | 1.5 |
| 27 | 23.860 | 3.72630 | 3.8 |
| 28 | 24.446 | 3.63835 | 1.4 |
| 29 | 25.385 | 3.50586 | 0.5 |
| 30 | 25.657 | 3.46927 | 0.4 |
| 31 | 26.497 | 3.36122 | 0.2 |
| 32 | 28.506 | 3.12872 | 1.2 |
| 33 | 36.110 | 2.48539 | 0.8 |
| 34 | 36.809 | 2.43979 | 0.4 |

As a preferred embodiment, the crystal form C has an X-ray powder diffraction pattern substantially as shown in FIG. 21.

In another aspect, the crystal form C further has a thermogravimetric analysis (TGA) pattern substantially as shown in FIG. 22 and/or a differential scanning calorimetry (DSC) pattern substantially as shown in FIG. 23.

All the X-ray powder diffraction patterns were measured using the Kα radiation of Cu target, unless otherwise stated.

Experimental temperatures in the present invention are all room temperature, unless otherwise stated. In some

embodiments, a method for preparing Compound 1 is provided.

In some embodiments, the method for preparing Compound 1 comprises the following steps:

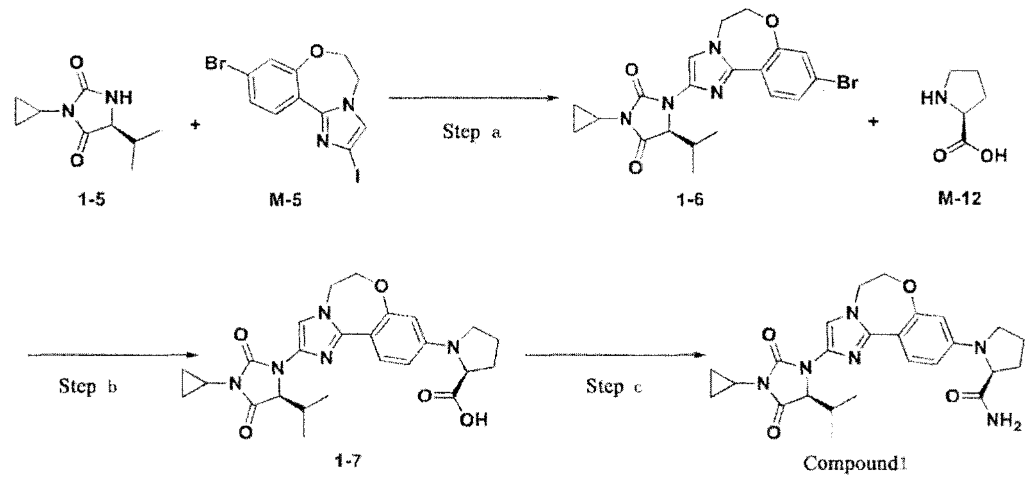


1-5 M-5 1-6 M-12

1-7 Compound1

a. obtaining Compound 1-6 by C-N coupling reaction of Compound 1-5 and Compound M-5 in the presence of a catalyst and a catalyst ligand under a basic condition;

b. obtaining Compound 1-7 by C-N coupling reaction of Compound 1-6 and Compound M-12 in the presence of a catalyst under a basic condition; and

c. forming Compound 1 by condensation reaction of Compound 1-7 and $NH_3$ in the presence of a condensing agent under a basic condition.

In some embodiments, the catalyst is not particularly limited, as long as the invention purpose of the present application can be achieved. For example, in step a, the catalyst may be selected from the group consisting of CuI, cuprous chloride, copper powder, cuprous oxide, copper acetate, copper acetylacetonate, copper sulfate and a combination thereof, preferably CuI or copper acetate; in step b, the catalyst may be selected from the group consisting of CuI, cuprous chloride, copper powder, cuprous oxide, cuprous thiophene-2-carboxylate and a combination thereof, preferably CuI.

In some embodiments, the catalyst ligand is not particularly limited, as long as the invention purpose of the present application can be achieved. For example, in step a, the catalyst ligand may be selected from the group consisting of trans-*N,N'*-dimethylcyclohexan-1,2-diamine, o-phenanthroline, *N,N'*-dimethylethylenediamine and a combination thereof, preferably trans-*N,N'*-dimethylcyclohexan-1,2-diamine or o-phenanthroline.

In some embodiments, the condensing agent is not particularly limited, as long as the invention purpose of the present application can be achieved. For example, in step c, the condensing agent may be selected from the group consisting of HATU, HBTU, PyBOP, BOP, DCC/HOBT, EDCI/HOBT, EDCI/HOSu, T3P, CDI, chloroformate, MsCl, TsCl, NsCl, Boc2O and a combination thereof, preferably HATU or HBTU. In some embodiments, the base used in the basic conditions is not particularly limited, as long as the invention purpose of the present application can be achieved. For example, in step a, the base may be selected from the group consisting of sodium carbonate, potassium carbonate, cesium carbonate, potassium phosphate, sodium bicarbonate, potassium bicarbonate, potassium hydrogen phosphate, sodium phosphate and a combination thereof, preferably potassium carbonate, potassium phosphate or cesium carbonate; in step b, the base may be selected from the group consisting of sodium carbonate, potassium carbonate, cesium carbonate, potassium phosphate, sodium bicarbonate, potassium bicarbonate, potassium hydrogen phosphate, sodium phosphate and a combination thereof, preferably potassium phosphate, potassium carbonate or cesium carbonate; in step c, the base may be selected from the group consisting of NMM, DIPEA and TEA, preferably NMM or TEA.

In some embodiments, the reactions of step a, step b and step c are each independently carried out in a reaction solvent, and none of the reaction solvents of step a, step b and step c are particularly limited, as long as the invention purpose of the present application can be achieved. For example, in step a, the solvent may be selected from the group consisting of DMF, acetonitrile, tetrahydrofuran, toluene, 2-MeTHF, ethylene glycol dimethyl ether and a combination thereof, preferably DMF and/or 2-MeTHF; in step b, the solvent may be selected from the group consisting of DMF, DMSO, NMP, THF and a combination thereof, preferably DMSO and/or DMF; and in step c, the solvent may be selected from the group consisting of DCM, DMSO, isopropanol, THF, ethyl acetate and a solvent mixture thereof, preferably DCM and/or DMSO.

In some embodiments, step a, step b and step c are each carried out at a certain reaction temperature, and

the reaction temperatures are not particularly limited, as long as the invention purpose of the present application can be achieved. For example, in step a, the reaction temperature may be 60°C to 130°C, preferably 70°C to 90°C; in step b, the reaction temperature may be 60°C to 130°C, preferably 80°C to 110°C; and in step c, the reaction temperature may be -5°C to 25°C, preferably 0°C to 20°C.

In some embodiments, Compound 1-7 may form a corresponding ammonium salt 1-7B with $NH_3$ in a solvent. In some embodiments, the solvent may be selected from the group consisting of ethyl acetate, 2-MeTHF, THF, dioxane, methanol and a combination thereof.

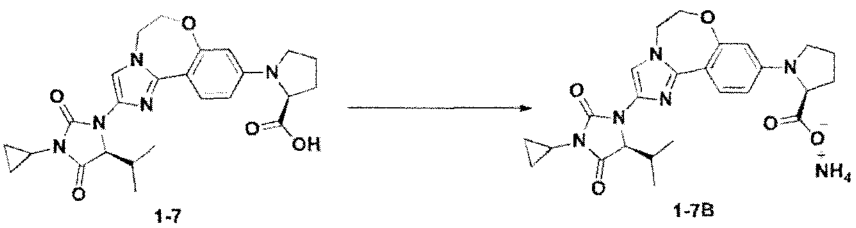

1-7 1-7B

In some embodiments, Compound 1-7B may form Compound 1 via a condensation reaction with $NH_3$ in the presence of a condensing agent.

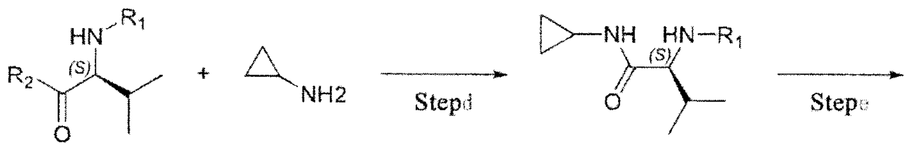


1-a 1-2 1-3

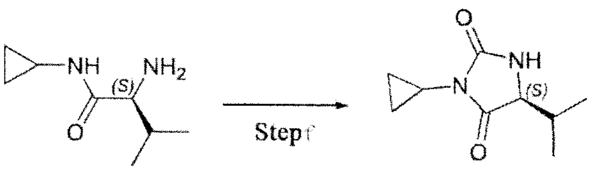


1-4 1-5

Step d: subjecting Compound 1-a and Compound 1-2 to a substitution reaction or a condensation reaction in the presence of a condensing agent to form Compound 1-3;

Step e: subjecting Compound 1-3 to a deprotection reaction under an acidic or basic condition or in the presence of a catalyst to obtain Compound 1-4 or a corresponding salt thereof;

Step f: subjecting Compound 1-4 to a ring-closing reaction in the presence of a ring-closing reagent to form Compound 1-5;

wherein, $R_1$ is a protecting group that can be selected from the group consisting of -Boc, -Cbz, -Fmoc, and trifluoroacetyl, preferably -Boc;

$R_2$ is selected from the group consisting of -Cl, imidazolyl and $-OR_3$;

$R_3$ is selected from the group consisting of H, $-C_{1-4}$ alkyl, phenyl, nitro-substituted phenyl, -C(O)-O-$C_{1-4}$ alkyl, -C(O)-O-phenyl and -C(O)-O-nitro-substituted phenyl.

In some embodiments, the condensing agent is not particularly limited, as long as the invention purpose of the present application can be achieved. For example, in step d, the condensing agent may be selected from the group consisting of HATU, HBTU, PyBOP, BOP, DCC/HOBT, EDCI/HOBT, EDCI/HOSu, T3P, CDI, chloroformate, MsCl, TsCl, NsCl, $Boc_2O$ and a combination thereof, preferably HATU.

In some embodiments, the ring-closing agent is not particularly limited, as long as the invention purpose of the present application can be achieved. For example, in step e, the ring-closing reagent may be selected from the group consisting of triphosgene, CDI, phenyl chloroformate, p-nitrophenyl chloroformate and a combination thereof.

In some embodiments, the reactions of step d and step f are each independently carried out in a reaction solvent, and none of the reaction solvents of step d and step f are particularly limited, as long as the invention purpose of the present application can be achieved. For example, in step d, the reaction solvent may be selected from the group

consisting of DCM, DMF and a solvent mixture thereof in any ratio; and in step f, the reaction solvent may be selected from the group consisting of DCM, THF, 2-MeTHF, acetonitrile, DMF and a combination thereof.

In some embodiments, the acidic conditions or basic conditions are not particularly limited, as long as the invention purpose of the present application can be achieved. For example, in step d, the base used in the basic condition may be selected from the group consisting of NMM, DIPEA and TEA; in step e, the base used in the basic condition may be selected from the group consisting of diethylamine and piperidine, preferably diethylamine; in step f, the base used in the basic condition may be selected from the group consisting of NMM, DIPEA, sodium bicarbonate, TEA and a combination thereof; and in step e, the acid used in the acid condition may be selected from the group consisting of TFA, $H_2SO_4$, HCl, $HBF_4$ and a combination thereof, preferably HCl.

In some embodiments, the catalyst is not particularly limited, as long as the invention purpose of the present application can be achieved. For example, in step e, the catalyst may be selected from the group consisting of $H_2$/Pd-C and $HCOONH_4$/Pd-C, preferably $H_2$/Pd-C. In some embodiments, there is provided a method 1 for preparing crystal form C of Compound 1, which comprises the following steps:

adding crystal form A of Compound 1 to a solvent selected from the group consisting of ethyl acetate, methanol, methanol/water mixture, n-butanol, methyl isobutyl ketone, isopropyl acetate, methyl tert-butyl ether and a combination thereof, and suspending and stirring the resultant at room temperature for 1 day to 5 days to obtain crystal form C of Compound 1.

In some embodiments, the method 1 further comprises the following steps: dissolving Compound 1 in a solvent mixture of methanol and dichloromethane until clear, precipitating a solid by slow dropwise addition of an antisolvent, and drying the solid to give crystal form A of Compound 1.

In some embodiments, there is provided a method 2 for preparing crystal form C of Compound 1, which comprises the following steps: dissolving Compound 1 in ethyl acetate, a solvent mixture of ethyl acetate and dimethyl sulfoxide, or a solvent mixture of methanol and ethyl acetate by heating until clear, slowly and dropwise adding an antisolvent until a solid is precipitated, cooling down the system to room temperature, filtering, and drying to give crystal form C of Compound 1. The antisolvent is selected from the group consisting of n-heptane, methyl tert-butyl ether, isopropyl ether and ethyl ether, preferably methyl tert-butyl ether. The various crystal forms of Compound 1 provided in the present invention have excellent cell proliferation inhibitory activity and superior anti-tumor effects. It was surprisingly found that the advantageous anhydrous crystal form further has better pharmacokinetic properties than existing PI3K$\alpha$ inhibitors (e.g., Example 102 in WO2017001658 or Example 101 in WO2017001645).

In some embodiments, there is provided a pharmaceutical composition comprising a therapeutically effective amount of the polymorph of Compound 1 or a mixture thereof of the present application and a pharmaceutically acceptable excipient, adjuvant or carrier.

In some embodiments, preferred embodiments of the above pharmaceutical composition are also provided.

As a preferred embodiment, the pharmaceutical composition is for oral administration.

As a preferred embodiment, the pharmaceutical composition is for use in tablets or capsules.

As a preferred embodiment, the pharmaceutical composition contains 0.01 wt% to 99 wt% of the crystal form of the present application.

As a preferred embodiment, the pharmaceutical composition contains 0.01 wt% to 20 wt% of the crystal form of the present application.

As a preferred embodiment, the pharmaceutical composition contains 1 wt% to 10 wt% of the crystal form of the present application.

In some embodiments, there is provided use of the crystal form or pharmaceutical composition in the preparation of a medicament.

In some embodiments, preferred technical solutions for the use are provided.

As a preferred embodiment, the medicament is for use in treating, preventing, delaying or stopping the onset or progression of cancer or cancer metastasis.

As a preferred embodiment, the medicament is for use as a PI3K inhibitor.

As a preferred embodiment, the medicament is for use in the treatment of a PI3K-mediated disease.

As a preferred embodiment, the PI3K is PI3K$\alpha$.

As a preferred embodiment, the PI3K-mediated disease is cancer.

As a preferred embodiment, the cancer is selected from the group consisting of sarcoma, prostate cancer, breast cancer, pancreatic cancer, lung cancer, gastrointestinal cancer, colorectal cancer, thyroid cancer, liver cancer, head and neck cancer, adrenal cancer, glioma, endometrial carcinoma, melanoma, kidney cancer, bladder cancer, uterine cancer, vaginal cancer, ovarian cancer, multiple myeloma, esophageal cancer, leukemia, brain cancer, oropharyngeal carcinoma, laryngocarcinoma, lymphoma, basal cell carcinoma, polycythemia vera and essential thrombocythemia.

In some embodiments, there is provided a method for treating and/or preventing a PI3K-mediated disease in patent, wherein the method is administering to a subject in need a therapeutically effective amount of an anhydrous crystal form or a solvate of Compound 1, or a pharmaceutical composition comprising an anhydrous crystal form or a

solvate of Compound 1.

As a preferred embodiment, the PI3K in the above method includes PI3Kα, PI3Kβ, PI3Kδ and/or PI3Kγ.

As a preferred embodiment, the PI3K in the above method is PI3Kα.

As a preferred embodiment, the PI3K-mediated disease in the above method is cancer.

As a preferred embodiment, the cancer in the above method is sarcoma, prostate cancer, breast cancer, pancreatic cancer, lung cancer, gastrointestinal cancer, colorectal cancer, thyroid cancer, liver cancer, head and neck cancer, adrenal cancer, glioma, endometrial carcinoma, melanoma, kidney cancer, bladder cancer, uterine cancer, vaginal cancer, ovarian cancer, multiple myeloma, esophageal cancer, leukemia, brain cancer, oropharyngeal carcinoma, laryngocarcinoma, lymphoma, basal cell carcinoma, polycythemia vera, or essential thrombocythemia.

In some embodiments, there is provided a method for treating cancer, wherein the method is administering to a subject in need a therapeutically effective amount of an anhydrous crystal form or a solvate of Compound 1 or a pharmaceutical composition comprising an anhydrous crystal form or a solvate of Compound 1, and the cancer is sarcoma, prostate cancer, breast cancer, pancreatic cancer, lung cancer, gastrointestinal cancer, colorectal cancer, thyroid cancer, liver cancer, head and neck cancer, adrenal cancer, glioma, endometrial carcinoma, melanoma, kidney cancer, bladder cancer, uterine cancer, vaginal cancer, ovarian cancer, multiple myeloma, esophageal cancer, leukemia, brain cancer, oropharyngeal carcinoma, laryngocarcinoma, lymphoma, basal cell carcinoma, polycythemia vera, or essential thrombocythemia.

As a preferred embodiment, the subject in need in the above method is human.

All crystal forms herein are substantially pure.

The term "substantially pure" as used herein means that the content by weight of the crystal form is not less than 85%, preferably not less than 95%, more preferably not less than 98%.

The term "solid form" and related terms used herein refer to a physical form that is not predominantly in liquid or gaseous state, and includes semisolid, if not otherwise specified. The solid form may be crystal form, amorphous form, disordered crystal form, partially crystal form, partially amorphous form or a mixture thereof.

The terms "crystal form", "crystalline form" and related terms can be used interchangeably herein to refer to a crystalline solid form, if not otherwise specified. Crystal form includes single-component and multi-component crystal forms, including, but not limited to, a solvent-free form (e.g., anhydrous crystal form), a solvate, a hydrate, a cocrystal, other molecular complexes, and a polymorph thereof, as well as a salt, a solvate, a hydrate, a cocrystal, other molecular complexes of the salt, and a polymorph thereof. In some embodiments, the crystal form of the material may be substantially free of amorphous and/or other crystal forms. In some embodiments, the crystal form of the material can contain about 50% or less by weight of one or more amorphous and/or other crystal forms. In some embodiments, the crystal form of the material may be physically and/or chemically pure.

The terms "polymorph", "polycrystalline form", "polycrystalline" and related terms as used herein refer to two or more crystal forms of a solid material substantially consisting of the same molecules and/or ions, if not otherwise specified. As with different crystal forms, different polymorphs may have different chemical and/or physical properties, including, but not limited to, for example, stability, solubility, dissolution rate, optical properties, melting point, mechanical properties, and/or density, and the like. These properties such as stability, dissolution and/or bioavailability of the medicament can affect the processing and/or manufacturing of the API. Accordingly, the polymorphism can affect at least one property of the medicament, including, but not limited to, quality, safety, and/or efficacy.

The term "solvate" as used herein refers to a molecular complex comprising API and a stoichiometric or non-stoichiometric amount of a solvent molecule, wherein the API may be a free base, a pharmaceutically available salt thereof, a cocrystal, a cocrystal of the salt, or other molecular complexes, if not otherwise specified. When the solvent is water, the solvate is "hydrate".

The term "amorphous form" as used herein refers to a disordered solid form of molecules and/or ions, which is not crystalline, if not otherwise specified. The amorphous form does not show a defined X-ray diffraction pattern with sharp defined peaks. Where not otherwise specified, Compound 1 is intended to cover any single solid form or a mixture of various solid forms of the free base.

Where not otherwise specified, the term "anhydrous crystal form" as used herein refers to a crystal form that does not contain water and other non-aqueous solvents.

As used herein, "anhydrous crystal form C", "crystal form C", "crystal form C of Compound 1" and the like can be used interchangeably; "anhydrous crystal form B-1", "crystal form B-1", "crystal form B-1 of Compound 1" and the like can be used interchangeably; and "crystal form A" and "crystal form A of Compound 1" can be used interchangeably.

As used herein, the terms "about" and "substantially" used in "has an X-ray powder diffraction pattern substantially as shown in FIG. 1" or "the X-ray powder diffraction pattern thereof is substantially as shown in FIG. 1" indicate that the exact positions of the peaks in the accompanying drawings should not be interpreted as absolute values. Since it is known to those skilled in the art that the 2θ value of the X-ray powder diffraction pattern may be subject to error due to different measurement conditions (e.g., equipments and instruments used) and different samples (e.g., different batches of samples), and the error in the measurement of the diffraction angle of the X-ray powder diffraction pattern

can be 5% or less. A difference of ±0.2° of a given value is generally considered to be appropriate. It should also be understood that the relative intensity of the peaks may fluctuate with experimental conditions and sample preparation such as the preferential orientation of particles in the sample. The use of automatic or fixed divergence slits can also affect the calculation of relative intensities. The intensities shown in the XRD curves included herein are only exemplary and cannot be used for absolute comparisons.

It will be understood by those skilled in the art that data measured by DSC and/or TGA may vary somewhat due to variations in sample purity, sample preparation and measurement conditions (e.g., instruments, heating rate). It should be understood that alternative heat absorption/melting point readings may be given by other kinds of instruments or by using different conditions from those described below. Accordingly, the DSC and/or TGA pattern cited in this application should not be taken as absolute values, and such measurement errors should be taken into account when interpreting the DSC and/or TGA data. The term "therapeutically effective amount" as used herein refers to the amount of a compound that, when administered to a subject in need for treating at least one clinical symptom of a disease or condition, is sufficient to affect such treatment of the disease, condition or symptom. The "therapeutically effective amount" may vary with the compound, the disease, condition and/or symptom of the disease or condition, the severity of the disease, condition and/or symptom of the disease or condition, the age of the patient being treated, and/or the weight of the patient being treated. In any particular case, a suitable amount may be apparent to those of skill in the art or may be determined using routine experiments. In the case of combination therapy, "therapeutically effective amount" means the total amount of effectively treating the disease, condition, or symptom of the subject in combination therapy.

The anhydrous crystal form and/or solvate described herein may be active components of a drug combination, which are mixed with a pharmaceutical carrier to form a pharmaceutical composition. The pharmaceutical carriers may be in various forms, depending on the mode of administration desired, e.g., oral or injectable (including intravenous) administration. Thus, the pharmaceutical composition of the present application may take the form of individual units suitable for oral administration, for example, capsules, cachets or tablets comprising a predetermined dose of the active component. Further, the pharmaceutical composition of the present application may be in the form of powders, granules, solutions, aqueous suspensions, non-aqueous liquids, oil-in-water emulsions or water-in-oil emulsions. Alternatively, the salt forms or crystal forms described in the present application may also be administered by means of a controlled release and/or delivery device, in addition to the common dosage forms mentioned above. The pharmaceutical composition of the present application can be prepared by any pharmaceutical method. Generally, the method comprises the step of associating the active component and the carrier constituting one or more of necessary components. Generally, the pharmaceutical composition is prepared by uniformly fully mixing the active component with a liquid carrier or a finely divided solid carrier or a mixture of both. In addition, the product can be easily prepared to the desired appearance.

"Pharmaceutically acceptable carrier" means a conventional pharmaceutical carrier suitable for a desired pharmaceutical formulation, e.g. diluent, excipient such as water, various organic solvents, and the like; filler such as starch slurry, pregelatinized starch, sucrose, dextrin, mannitol, lactose, spray-dried lactose, microcrystalline cellulose, silicified microcrystalline cellulose, inorganic salts, and the like; binder such as starch slurry, dextrin, powdered sugar, syrup, gum slurry, polyethylene glycol, cellulose derivatives, alginate, gelatin, hydroxypropyl cellulose, copovidone, polyvinylpyrrolidone (PVP), and the like; wetting agent such as distilled water, ethanol, glycerol, and the like; disintegrating agent such as dry starch, low-substituted hydroxypropyl cellulose, hydroxypropyl starch, agar, calcium carbonate, sodium bicarbonate, crosslinked povidone, crosslinked sodium carboxymethyl cellulose, sodium carboxymethyl starch, and the like; absorption enhancer such as quaternary ammonium compounds, amino acid ethylamine derivatives, acetoacetates, beta-dicarboxylic acid esters, aromatic acidic compounds, aliphatic acidic compounds, and the like; surfactant such as sodium cetyl sulphate, sodium octadecyl sulphate, sodium dioctyl sulfosuccinate, sodium dodecyl sulfonate, benzalkonium bromide, benzalkonium chloride, duromifene, lecithin, cetyl alcohol, sodium dodecyl sulphate, tween, Span, and the like; drug-carrying matrix such as polyethylene glycol, carbomer, cellulose derivatives, glycerol gelatin, polyvinyl alcohol, cocoa bean esters, synthetic or fully synthetic fatty acid glycerides, polyvinyl alcohol 40 stearate, vaseline, solid paraffin wax, liquid paraffin wax, dimethyl silicone oil, lanolin, beeswax, adeps suillus, and the like; absorbent carrier such as kaolin, bentonite, and the like; lubricant such as talc, aerosil, silicon dioxide, hydrogenated vegetable oils, magnesium dodecyl sulphate, sodium dodecyl sulphate, stearic acid, calcium stearate, magnesium stearate, sodium stearate fumarate, polyethylene glycol, and the like. Further, other pharmaceutically acceptable excipients such as antioxidants, colorants, preservatives, pH modifiers, hardeners, emulsifiers, propellants, dispersants, stabilizers, thickeners, complexing agents, buffers, permeation enhancers, polymers, aromatics, sweeteners, and dyes may also be added to the pharmaceutical composition. An excipient suitable for the desired dosage form and desired mode of administration is preferred.

The term "disease" or "condition" or "symptom" refers to any illness, discomfort, disease, symptom or indication.

## Examples

The following examples are set forth for fully understanding the present invention. These examples are used

to illustrate exemplary embodiments of the present invention and should not be construed as limiting the scope of the present invention in any way. It will also be understood by those skilled in the art that the present invention can be implemented without using these details. The headings used throughout the present disclosure are for convenience only and are not to be construed as limiting the claims in any way. Embodiments described under any heading may be combined with embodiments described under any other heading.

The reactants used in the following examples may be obtained as recited herein or, if not recited herein, are commercially available or may be prepared from commercially available materials by methods known in the art. Unless otherwise noted, it is easy for those skilled in the art to select achievable solvents, temperatures, pressures, reactant ratios, and other reaction conditions. Typically, the reaction process can be monitored by thin-layer analysis or high-pressure liquid chromatography (HPLC), and the intermediates and products can be purified by silica gel chromatography, salt formation and/or recrystallisation, if desired.

## Instruments

### X-ray Powder Diffraction (XRPD)

Data was acquired on a Bruker X-ray powder diffractometer (Bruker D8 Advance) using a Lynxeye detector. Acquisition conditions were: radiation: Cu K$\alpha$, generator voltage: 40 kV, generator current: 40 mA, start angle: 3.0° 2$\theta$, end angle: 40.0° 2$\theta$, step size: 0.02° 2$\theta$, and scanning time per step: 0.2 seconds, 0.3 seconds, or 0.6 seconds. During the sample preparation, an appropriate amount of sample was placed onto the background-free wafer sample tray and flatten to ensure that the sample surface was smooth and flat.

### Differential Scanning Calorimetry (DSC)

DSC thermal analysis patterns were obtained using a TA Instrument Discovery DSC 2500 Differential Scanning Calorimeter at a heating rate of 10°C/min under $N_2$ purge at 50 ml/min.

### Thermogravimetric Analysis (TGA)

TGA thermal analysis patterns were obtained using a TA Instrument Discovery TGA 550 thermogravimetric analyzer at a heating rate of 10°C/min under $N_2$ purge at 40ml/min.

### Nuclear Magnetic Resonance Hydrogen Spectroscopy ($^1$H-NMR)

$^1$H-NMR (Bruker AVANCE NEO 500): $^1$H-NMR spectra were recorded using a 500 MHz proton frequency, excitation pulse, and a 1 s cyclic delay. A total of 16 scans were performed and deuterated DMSO was used as solvent. The solvent peak was used as a reference and chemical shifts were reported on the TMS scale.

### Dynamic Vapor Sorption (DVS)

A Surface Measurement Systems Dynamic Vapor Sorption Analyzer (model of DVS Resolution) was used with an $N_2$ flow rate: 200 sccm, detection temperature: 25°C, equilibrium time range: 5 min to 360 min, RH gradient: 10% (50%RH to 90%RH, 90%RH to 0%RH to 90%RH), and 5% (90%RH to 95%RH, 95%RH to 90%RH) to obtain dynamic vapor sorption analysis pattern.

### Single Crystal X-ray Diffraction

Diffraction intensity data was collected with a Bruker D8 Venture Photon II diffractometer with CuK$\alpha$ radiation as the light source and scanning mode: $\varphi/\omega$ scan.

## Abbreviations:

API: active pharmaceutical ingredient;
Boc: tert-butoxycarbonyl;
$Cs_2CO_3$: cesium carbonate;
CDI: N,N'-carbonyl diimidazole;

CuI: cuprous iodide;
DSC: Differential Scanning Calorimetry;
DVS: Dynamic Moisture Sorption;
DCM: dichloromethane;
DMF: N,N-dimethylformamide;
DMSO: dimethyl sulfoxide;
EA: ethyl acetate;
EDCI: 1-(3-dimethylaminopropyl)-3-ethylcarbodiimide hydrochloride;
hrs/h: hour(s);
HPLC: High Performance Liquid Chromatography;
HOBT: 1-hydroxybenzotriazole;
HATU: 2-(7-azobenzotriazole)-N,N,N',N'-tetramethyluronium hexafluorophosphate;
IPAc: isopropyl acetate;
LCMS/LC-MS: Liquid Chromatography-Mass Spectrometry;
min: minute(s);
MIBK: methyl isobutyl ketone;
MTBE: methyl tert-butyl ether;
MeOH: methanol;
NMM: N-methylmorpholine;
NIS: N-iodosuccinimide;
PE: petroleum ether;
Pre-HPLC: Preparative High Performance Liquid Chromatography;
RH: relative humidity;
rt or RT: room temperature;
RRT: relative retention time;
Solutol: polyethylene glycol 15-hydroxystearate
TEA: triethylamine;
THF: tetrahydrofuran;
TGA: Thermogravimetric Analysis;
$^1$H-NMR/HNMR/$^1$H NMR: Nuclear Magnetic Resonance Hydrogen Spectroscopy;
XRD/XRPD: X-ray Powder diffraction.
BOP: benzotriazol-1-yloxytris(dimethylamino)phosphonium hexafluorophosphate;
Cbz: benzyloxycarbonyl;
DIPEA: *N,N*-diisopropylethylamine;
EtOH: ethanol;
Fmoc: 9-fluorenylmethoxycarbonyl;
HBTU: benzotriazol-N,N,N',N'-tetramethylurea hexafluorophosphate;
HOSu: N-hydroxysuccinimide;
Hex: n-hexane;
$K_2CO_3$: potassium carbonate;
$K_3PO_4$: potassium phosphate;
MsCl: methylsulfonyl chloride;
NMP: N-methylpyrrolidone;
NsCl: p-nitrobenzenesulfonyl chloride;
T3P: 1-propylphosphonic anhydride;
TFA: trifluoroacetic acid;
TsCl: p-toluenesulfonyl chloride;
2-MeTHF: 2-methyltetrahydrofuran.

**Preparation of Intermediate M-5**

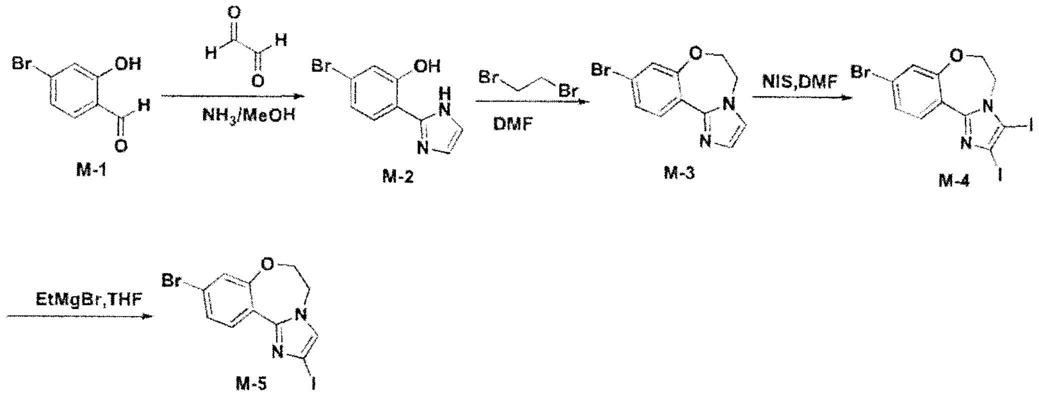


**Step 1: Preparation of Compound M-2**

4-Bromo-2-hydroxybenzaldehyde (40 g) and MeOH (400 mL) were added to a 1000 mL single-necked flask, and aqueous ammonia (136.50 g) was added dropwise with stirring in an ice bath. The system was heated in an oil bath at 35°C. The reaction was monitored by LC-MS until completion. The reaction solution was concentrated, diluted with water, and extracted four times using EA. The organic phases were combined, dried, concentrated, purified by column chromatography (PE:EA=3:1), and concentrated to obtain 34.55 g of Compound M-2.
LC-MS $[M+H]^+$: 239.

Step 2: Preparation of Compound M-3

Compound M-2 (34.55 g), $Cs_2CO_3$ (133 g) and DMF (300 mL) were added to a 1000 mL single-necked flask with stirring at room temperature for 20 min, and then 1,2-dibromoethane (54.30 g) was added dropwise. After the dropwise addition, the reaction solution was refluxed in an oil bath at 80°C, and the reaction was monitored by LC-MS until completion. The reaction solution was concentrated, diluted with water, and extracted three times using EA. The organic phases were combined, dried, concentrated under reduced pressure, purified by column chromatography (PE:EA=70:30), and concentrated to obtain 21.37 g of Compound M-3.
LC-MS $[M+H]^+$: 265.
$^1H$ NMR (500 MHz, chloroform-d) δ 8.38 (d, J = 8.5 Hz, 1H), 7.30-7.15 (m, 3H), 6.99 (s, 1H), 4.47-4.43 (m, 2H), 4.41 -4.35 (m, 2H).

**Step 3: Preparation of Compound M-4**

Compound M-3 (21.37 g) and DMF (100 mL) were added to a 1000 mL single-necked flask with stirring until dissolution, and then a solution of NIS (50.78 g) in DMF (100 mL) was added dropwise. After the dropwise addition, the reaction was carried out in an oil bath at 60°C overnight, and was monitored by LC-MS until completion. Water was added to the reaction solution to precipitate a solid, which was filtered and dried to obtain 35 g of Compound M-4.
LC-MS $[M+H]^+$: 517.
$^1H$ NMR (500 MHz, chloroform-*d*) δ 8.30 (d, J = 8.6 Hz, 1H), 7.25-7.16 (m, 2H), 4.46 -4.41 (m, 2H), 4.36-4.32 (m, 2H).

**Step 4: Preparation of Compound M-5**

Compound M-4 (35 g) and THF (150 mL) were added into a 500 mL three-necked flask, and 100 mL of ethyl magnesium b romide (1 M THF solution) was dropwise added to the reaction system at -40°C under nitrogen protection. After the dropwise addition, the reaction solution was stirred at -40°C. The reaction was monitored by LC-MS until completion. Saturated ammonium chloride solution was added to quench the reaction in an ice bath, and EA was added to extract for three times. The organic phases were combined, dried, concentrated, slurried by adding methyl tert-butyl ether, filtered and dried to obtain 19.8 g of Compound M-5.
LC-MS $[M+H]^+$: 391.
$^1H$ NMR (500 MHz, DMSO-d6) δ 8.22 (d, J = 8.6 Hz, 1H), 7.55 (s, 1H), 7.31 - 7.23 (m, 2H), 4.47 -4.39 (m, 4H).

**Example 1 Preparation of (*S*)-1-(2-(3-cyclopropyl-5-isopropyl-2,4-dioxoimidazolidin-1-yl)-5,6-dihydrobenzo[*f*]imidazo[1,2-*d*][1,4]oxazepin-9-yl)pyrrolidine-2-carboxamide (Compound A)**

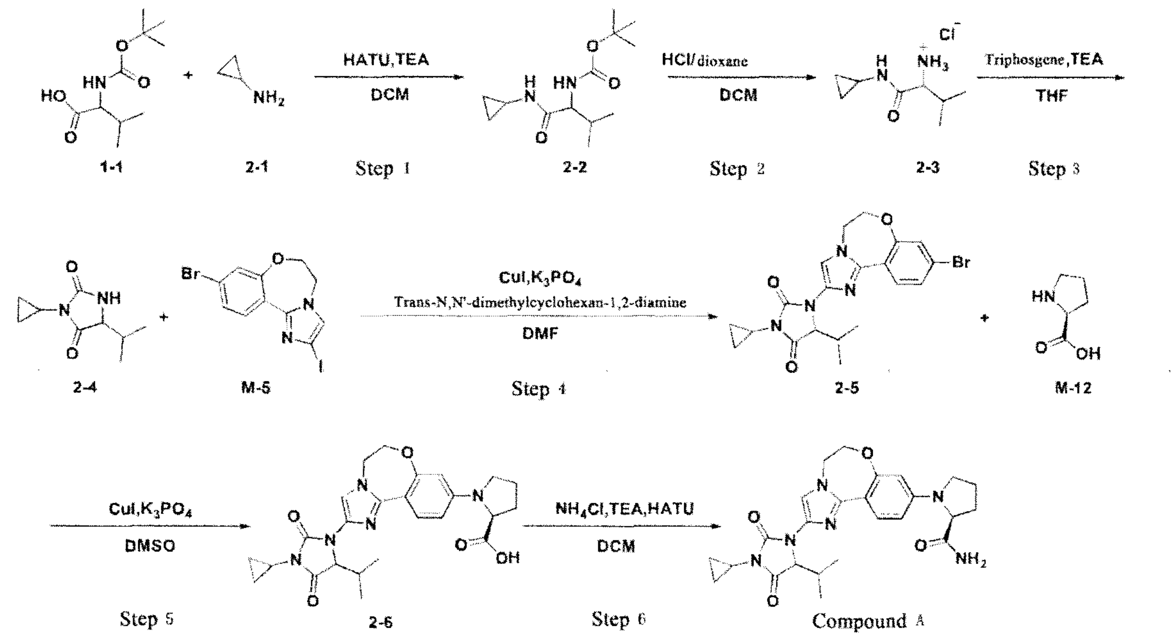

1-1 2-1 2-2 2-3 2-4 M-5 2-5 M-12 2-6 Compound A

**Step 1: Preparation of Compound 2-2**

Compound 1-1 (5.0 g), DCM (100 mL) and HATU (9.55 g) were added into a 250 mL single-necked flask under the protection of nitrogen. TEA(8.15 g) and Compound 2-1 (1.45 g) were then added in an ice bath, and the reaction was carried out at room temperature for 2 h. After completion of the reaction was monitored by LCMS, the reaction solution was concentrated, diluted with EA, washed with water, dried with anhydrous sodium sulfate, and concentrated to obtain 5.0 g of Compound 2-2, which was directly used in the next step.

LC-MS[M-Boc+H]$^+$: 157.

$^1$H NMR (500 MHz, DMSO-d6) δ 7.91 (s, 1H), 6.54 (d, J=9.0 Hz, 1H), 3.69-3.58

(m, 1H), 2.65-2.55 (m, 1H), 1.88-1.80 (m, 1H), 1.37 (s, 9H), 0.79 (d, J=6.0 Hz, 6H), 0.62-0.58 (m, 2H), 0.42-0.30 (m, 2H).

**Step 2: Preparation of Compound 2-3**

Compound 2-2 (5 g), DCM (20 mL) and HCl/dioxane (20 mL, 4.0 mol/L) were added to a 250 mL single-necked flask, and the reaction was carried out at room temperature for 2 h. After completion of the reaction was monitored by LCMS, the reaction solution was concentrated to obtain 3.75 g of Compound 2-3.

LC-MS [M+H]$^+$: 157.

**Step 3: Preparation of Compound 2-4**

Compound 2-3 (2.0 g), DCM (50 mL) and TEA (4.20 g) were added to a 250 mL three-necked flask under the protection of nitrogen. A solution of triphosgene (1.54 g) in DCM (50 mL) was dropwise added to the reaction system in an ice bath, and the reaction was carried out with stirring in an ice bath for 2 h. After completion of the reaction was monitored by LCMS, the reaction was quenched with ice water in an ice bath. The reaction solution was concentrated, extracted by EA, dried with anhydrous $Na_2SO_4$, and concentrated. The crude product was purified by column chromatography (PE:EA=100:0 to 50:50) to obtain 810 mg of Compound 2-4.

LC-MS [M+H]$^+$: 183.

$^1$H NMR (500 MHz, chloroform-d) δ 6.28 (s, 1H), 3.85 (d, J = 3.6 Hz, 1H), 2.66 -2.48

(m, J = 3.7 Hz, 1H), 2.25-2.15 (m, 1H), 1.03 (d, J = 6.5 Hz, 3H), 0.98 -0.91 (m, 4H), 0.88 (d, J = 7.0 Hz, 3H).

**Step 4: Preparation of Compound 2-5**

Compound 2-4 (431 mg), Compound M-5 (700 mg), DMF (10 mL), CuI (102 mg), trans-N,N'-dimethylcyclohexan-1,2-diamine (77 mg) and $K_3PO_4$ (760 mg) were added to a 50 mL single-necked flask under the protection of nitrogen, and the reaction was heated and carried out at a temperature of 120°C for 2 h with stirring. After completion of the reaction was monitored by LCMS, the reaction solution was diluted by EA, washed with water, dried with anhydrous $Na_2SO_4$, and concentrated. The crude product was purified by column chromatography (PE:EA=100:0 to 60:40) to obtain 642 mg of Compound 2-5.

LC-MS $[M+H]^+$: 445/447.

$^1H$ NMR (500 MHz, chloroform-d) $\delta$ 8.23 (d, J = 8.5 Hz, 1H), 7.27 (s, 1H), 7.22 (d, J = 8.7 Hz, 1H), 7.20 (s, 1H), 4.63-4.59 (m, 1H), 4.51-4.38 (m, 2H), 4.36 (t, J = 4.3 Hz, 2H), 2.79-2.71 (m, 1H), 2.68-2.60 (m, 1H), 1.24 (d, J = 7.1 Hz, 3H), 1.02-0.94 (m, 4H), 0.81 (d, J = 6.9 Hz, 3H).

**Step 5: Preparation of Compound 2-6**

Compound 2-5 (642 mg), Compound M-12 (415 mg), $K_3PO_4$ (919 mg) and DMSO (10 mL) were added to a 30 mL microwave tube with nitrogen purge, to which CuI (83 mg) was added, and the reaction was heated and carried out at a temperature of 120°C for 1 h under microwave. After completion of the reaction was monitored by LCMS, the reaction solution was directly used in the next step.

LC-MS $[M+H]^+$: 480.

**Step 6: Preparation of Compound A**

The reaction solution obtained in step 5 was added to a 50 mL single-necked flask with nitrogen displacement, to which DCM (10 mL), $NH_4Cl$ (462 mg) and TEA (1.46 g) were added, and the reaction system was cooled down to 0°C. HATU (3.28 g) was added in an ice bath, and the reaction was carried out at 0°C for 1 h with stirring. After completion of the reaction was monitored by LCMS, the reaction solution was diluted by adding DCM and washed with water. The organic phase was dried with anhydrous $Na_2SO_4$ and concentrated. The resulting crude product was purified by Pre-HPLC (C18 column, $H_2O$:MeOH=95:5 to 50:50) to obtain 85 mg of Compound A, a mixture of a pair of diastereoisomers.

LC-MS $[M+H]^+$: 479.

$^1H$ NMR (500 MHz, DMSO-d6) $\delta$ 8.03 (d, J = 9.0 Hz, 1H), 7.40 (s, 1H), 7.24 (s, 1H), 7.05 (s, 1H), 6.32 (d, J = 8.9 Hz, 1H), 6.03 (d, J = 2.6 Hz, 1H), 4.48 (s, 1H), 4.41 -4.29 (m, 4H), 3.94 (d, J = 8.8 Hz, 1H), 3.55 (t, J = 8.1 Hz, 1H), 3.25-3.19 (m, 1H), 2.74 -2.55 (m, 2H), 2.25-2.14 (m, 1H), 2.07-1.85 (m, 3H), 1.13 (d, J = 7.0 Hz, 3H), 0.88 (d, J = 7.2 Hz, 2H), 0.81 (d, J = 3.9 Hz, 2H), 0.71 (d, J = 6.8 Hz, 3H).

**Example 2 Preparation of (*S*)-1-(2-((*S*)-3-cyclopropyl-5-isopropyl-2,4-dioxoimidazolidin-1-yl)-5,6-dihydrobenzo[*f*]imidazo[1,2-d][1,4]oxazepin-9-yl)pyrrolidine-2-carboxamide (Compound 1) and (*S*)-1-(2-((*R*)-3-cyclopropyl-5-isopropyl-2,4-dioxoimidazolidin-1-yl)-5,6-dihydrobenzo[*f*]imidazo[1,2-d][1,4]oxazepin-9-yl)pyrrolidine-2-carboxamide (Compound 2)**

In this example, Compound 1 (back peak) and Compound 2 (front peak) can be obtained by a chiral separation of Compound A on a chiral column under the following conditions.

The conditions of chiral HPLC:

| Chromatographic Column | CHIRALPAK IA |
|---|---|
| Specification of the Chromatographic Column | 3 cm×25 cm, 5 um |
| Sample Size | 4.0 mL |
| Mobile Phase | (Hex:DCM =3:1):EtOH=50:50 (v/v) |
| Flow Rate | 35 mL/min |

(continued)

| Chromatographic Column | CHIRALPAK IA |
|---|---|
| Wavelength | UV 220 nm |
| Temperature | 25°C |
| Sample Solution | 12.3 mg/mL of solution of EtOH:DCM=3:1 |
| Prep-HPLC apparatus | Prep-HPLC-flash |

Compound 2: LC-MS $[M+H]^+$: 479.

$^1H$ NMR (500 MHz, DMSO-d6) δ 8.03 (d, J = 9.0 Hz, 1H), 7.40 (s, 1H), 7.24 (s,

1H), 7.05 (s, 1H), 6.32 (d, J = 8.9 Hz, 1H), 6.03 (d, J = 2.6 Hz, 1H), 4.48 (s, 1H), 4.41 - 4.29 (m, 4H), 3.94 (d, J = 8.8 Hz, 1H), 3.55 (t, J = 8.1 Hz, 1H), 3.25-3.19 (m, 1H), 2.74 - 2.55 (m, 2H), 2.25-2.14 (m, 1H), 2.07-1.85 (m, 3H), 1.13 (d, J = 7.0 Hz, 3H), 0.88 (d, J = 7.2 Hz, 2H), 0.81 (d, J = 3.9 Hz, 2H), 0.71 (d, J = 6.8 Hz, 3H).

Compound 1: LC-MS $[M+H]^+$: 479.

$^1H$ NMR (500 MHz, DMSO-d6) δ 8.03 (d, J = 9.0 Hz, IH), 7.40 (s, 1H), 7.24 (s,

1H), 7.05 (s, 1H), 6.32 (d, J = 8.9 Hz, 1H), 6.03 (d, J = 2.6 Hz, 1H), 4.48 (s, 1H), 4.41 - 4.29 (m, 4H), 3.94 (d, J = 8.8 Hz, 1H), 3.55 (t, J = 8.1 Hz, 1H), 3.25-3.19 (m, 1H), 2.74 - 2.55 (m, 2H), 2.25-2.14 (m, 1H), 2.07-1.85 (m, 3H), 1.13 (d, J = 7.0 Hz, 3H), 0.88 (d, J = 7.2 Hz, 2H), 0.81 (d, J = 3.9 Hz, 2H), 0.71 (d, J = 6.8 Hz, 3H).

The obtained Compound 1 was structurally identified to have an XRPD pattern substantially as shown in FIG. 32, wherein the X-ray powder diffraction pattern thereof has characteristic peaks at 2θ of 6.1°±0.2°, 6.2°±0.2°, 7.3°±0.2°, 7.6°±0.2°, 9.1°±0.2°, 9.3°±0.2° and 17.2°±0.2°, and was defined as crystal form M. The identification result of the obtained Compound 2 showed that it was amorphous.

**Example 3 Preparation of Compound 1**

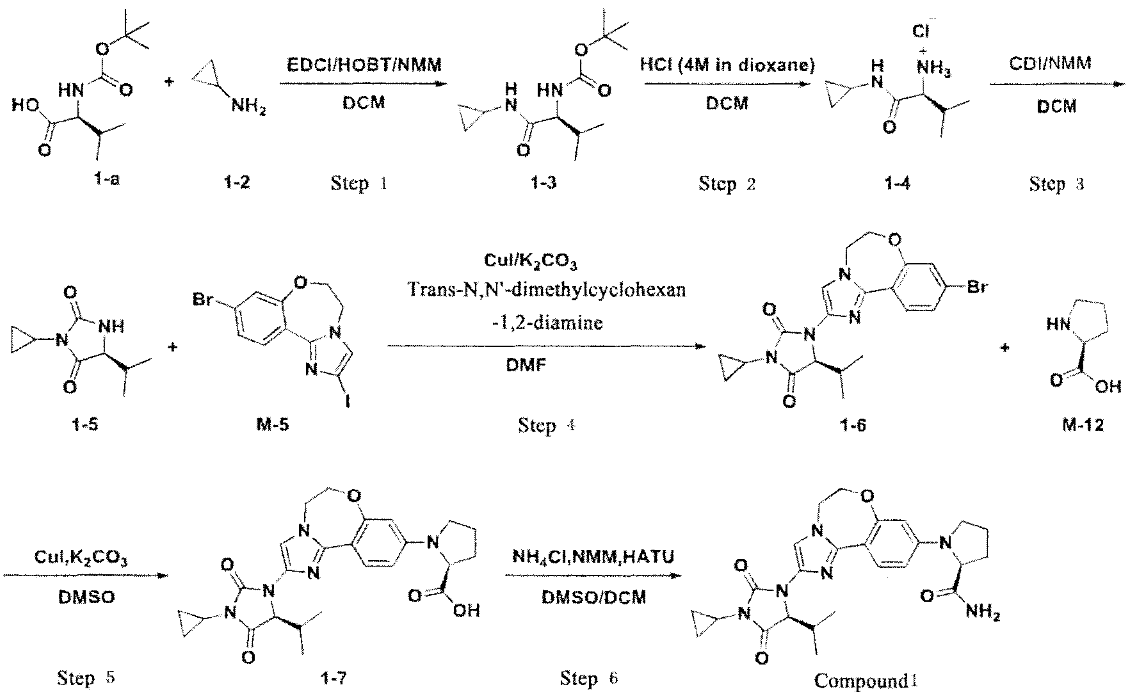


1-a 1-2 Step 1 1-3 Step 2 1-4 Step 3

1-5 M-5 Step 4 1-6 M-12

Step 5 1-7 Step 6 Compound1

**Step 1: Preparation of Compound 1-3**

Compound 1-a (257 g), DCM (2.5 L) and NMM (274 g) were added into a 5 L three-necked flask under the protection of nitrogen. EDCI (250 g), HOBT (200 g) and Compound 1-2 (74 g) were sequentially added in an ice bath. The reaction was carried out at room temperature overnight. After completion of the reaction was monitored by LCMS, the reaction solution was added with water and extracted with DCM. The organic phase was washed with saturated saline, dried, and concentrated under reduced pressure to obtain the residue, and the residue was slurried with MTBE to obtain 278 g of white solid.

LC-MS$[M\text{-}Boc+H]^+$: 157.

$^1$H NMR (500 MHz, DMSO-d6) δ 7.91 (s, 1H), 6.54 (d, J=9.0 Hz, 1H), 3.69-3.58

(m, 1H), 2.65-2.55 (m, 1H), 1.88-1.80 (m, 1H), 1.37 (s, 9H), 0.79 (d, J=6.0 Hz, 6H), 0.62-0.58 (m, 2H), 0.42-0.30 (m, 2H).

**Step 2: Preparation of Compound 1-4**

Compound 1-3 (278 g), DCM (1.0 L) and HCl/dioxane (1.0 L, 4.0 M/L) were added to a 3 L single-necked flask, and the reaction was carried out at room temperature overnight. After completion of the reaction was monitored by LCMS, the reaction solution was concentrated to obtain 209 g of Compound 1-4.

LC-MS $[M+H]^+$: 157.

**Step 3: Preparation of Compound 1-5**

Compound 1-4 (169 g), DCM (2.0 L) and NMM (329 g) were added to a 5 L three-necked flask under the protection of nitrogen. The system was heated to 50°C, added with CDI (229 g), and carried out at 50°C for 30 min. The reaction solution was cooled down and filtered. The filtrate was concentrated under reduced pressure, added with 2 M hydrochloric acid to adjust the pH to 2, and extracted with ethyl acetate. The organic phase was washed with saturated saline, dried, filtered and concentrated under reduced pressure to obtain a crude product, which was slurried with PE/EA, filtered and dried to obtain Compound 1-5 (100 g).

LC-MS $[M+H]^+$: 183.

$^1$H NMR (500 MHz, chloroform-d) δ 6.28 (s, 1H), 3.85 (d, J = 3.6 Hz, 1H), 2.66 -2.48

(m, J = 3.7 Hz, 1H), 2.25-2.15 (m, 1H), 1.03 (d, J = 6.5 Hz, 3H), 0.98 -0.91 (m, 4H), 0.88 (d, J = 7.0 Hz, 3H).

**Step 4: Preparation of Compound 1-6**

Compound 1-5 (6.1 g), Compound M-5 (13.09 g), DMF (131 mL), CuI (2.25 g), trans-N,N'-dimethylcyclohexan-1,2-diamine (1.90 g), molecular sieve (13.09 g) and $K_2CO_3$ (9.25 g) were added to a 500 mL single-necked flask under the protection of nitrogen, and the reaction was heated and carried out at a temperature of 85°C for 3 h with stirring. After completion of the reaction was monitored by LCMS, the reaction solution was diluted by EA, washed with water, dried with anhydrous $Na_2SO_4$, and concentrated, and a crude product was purified by column chromatography (PE:EA=100:0 to 60:40) to obtain 6.89 g of Compound 1-6.

LC-MS $[M+H]^+$: 445/447.

$^1H$ NMR (500 MHz, chloroform-d) δ 8.23 (d, J = 8.5 Hz, 1H), 7.27 (s, 1H), 7.22 (d, J =

8.7 Hz, 1H), 7.20 (s, 1H), 4.63-4.59 (m, 1H), 4.51-4.38 (m, 2H), 4.36 (t, J = 4.3 Hz, 2H), 2.79-2.71 (m, 1H), 2.68-2.60 (m, 1H), 1.24 (d, J = 7.1 Hz, 3H), 1.02-0.94 (m, 4H), 0.81 (d, J = 6.9 Hz, 3H).

**Step 5: Preparation of Compound 1-7**

Compound 1-6 (6 g), Compound M-12 (4.65 g), CuI (2.57 g), $K_2CO_3$ (3.72 g) and DMSO (180 mL) were added to a 500 mL single-necked flask with nitrogen displacement, and the reaction was heated and carried out at a temperature of 110°C for 1 h with stirring. After completion of the reaction was monitored by LCMS, the reaction solution was filtered. The filter cake was washed with 170 mL DCM, and the resulting filtrate was used directly in the next step.

LC-MS $[M+H]^+$: 480.

**Step 6: Preparation of Compound 1**

The filtrate obtained in step 5 was added to a 500 mL single-necked flask with nitrogen displacement, followed by the addition of $NH_4Cl$ (6.60 g) and NMM (12.49 g), and the reaction system was cooled down to 0°C. HATU (18.63 g) was added in an ice bath, and the reaction was carried out at 0°C for 1h with stirring. After completion of the reaction was monitored by LCMS, the reaction was quenched with water, extracted with ethyl acetate, and the organic phase was washed with saturated saline, dried with anhydrous $Na_2SO_4$ and concentrated. 34 mL of methanol and 54 mL of dichloromethane were added to the resulting crude product and heated until dissolution and clear. The resultant was cooled down naturally to room temperature, and the solid was precipitated by slow and dropwise addition of ether, which was filtered. The filter cake was washed with ether and dried to obtain 2.88 g of Compound 1.

Identification was carried out by X-ray powder diffraction, which showed that the crystal form of Compound 1 was crystal form A. The X-ray powder diffraction pattern was substantially as shown in FIG. 1.

LC-MS $[M+H]^+$: 479.

$^1H$ NMR (500 MHz, DMSO-d6) δ 8.03 (d, J = 9.0 Hz, 1H), 7.40 (s, 1H), 7.24 (s,

1H), 7.05 (s, 1H), 6.32 (d, J = 8.9 Hz, 1H), 6.03 (d, J = 2.6 Hz, 1H), 5.76 (s, 1.56H, DCM), 4.48 (s, 1H), 4.41 - 4.29 (m, 4H), 3.94 (d, J = 8.8 Hz, 1H), 3.55 (t, J = 8.1 Hz, 1H), 3.25-3.19 (m, 1H), 2.74 - 2.55 (m, 2H), 2.25-2.14 (m, 1H), 2.07-1.85 (m, 3H), 1.13 (d, J = 7.0 Hz, 3H), 0.88 (d, J = 7.2 Hz, 2H), 0.81 (d, J = 3.9 Hz, 2H), 0.71 (d, J = 6.8 Hz, 3H).

**Example 4 Preparation of the polymorph of Compound 1**

Free base Compound 1 was screened and evaluated for polymorph in a variety of ways and means such as room temperature/high temperature suspension stirring crystallization, gas-liquid permeation crystallization, gas-solid permeation crystallization, antisolvent addition crystallization, slow volatilization crystallization, and the like. Several polymorphs were screened and characterized, including (but not limited to) the following:

Crystal form A

Crystal form A is dichloromethane solvate, $^1H$-NMR results of which showed that about 12.00 wt% of dichloromethane solvent was contained in the crystal form A sample.

Crystal form B-1

Crystal form A was heated to 150°C, and then cooled to room temperature for XRPD characterization as shown in FIG. 18, indicating that anhydrous crystal form B-1 was obtained. The TGA and DSC results showed that no significant weight loss was observed for crystal form B-1 before 200°C, and an endothermic peak at around 186°C (peak temperature), one exothermic peak at around 189°C (peak temperature) and an endothermic peak at around 229°C (peak temperature) were observed.

Crystal form B-2

Crystal form A was placed in an acetonitrile atmosphere for gas-solid diffusion for 13 days to obtain crystal form B-2 as acetonitrile solvate, which was characterized by XRPD (FIG. 4) and $^1$H-NMR. The $^1$H-NMR results showed that the crystal form B-2 sample contained about 7 wt% of the acetonitrile solvent. The TGA/DSC results showed that the sample had a weight loss of about 5.14 wt% before 100°C, and three endothermic peaks at around 165°C, 167°C and 236°C, respectively, were observed.

Crystal form B-3

Crystal form A was placed in a dichloromethane atmosphere for gas-solid diffusion for 13 days to obtain crystal form B-3 as dichloromethane solvate, and the sample was characterized by XRPD (FIG. 5). The TGA/DSC results showed that the sample had a weight loss of about 4.91 wt% before 144.55°C, and an endothermic peak at around 191°C and an exothermic peak at around 236°C were observed. The XRPD pattern of crystal form B-3 was different from that of crystal form A, indicating the occurrence of a crystal form of dichloromethane solvate different from crystal form A.

Crystal form B-1 is anhydrous crystal form, crystal form B-2 is acetonitrile solvate, and crystal form B-3 is dichloromethane solvate. All the three crystal forms do not contain solvent or contain different solvents in their lattices, but the numbers and the positions of the diffraction peaks in the XRPD pattern are substantially the same. Therefore crystal form B-1, crystal form B-2, and crystal form B-3 are considered to have the isomorphic structure.

Crystal form D-1

The crystal form A sample was suspended and stirred in acetone for 1 day at room temperature to obtain crystal form D-1 as acetone solvate, which was characterized by XRPD (FIG. 6) and $^1$H-NMR. The $^1$H-NMR results showed that the crystal form D-1 sample contained about 5.62 wt% of acetone solvent. The TGA/DSC results showed that the sample had a weight loss of about 5.94 wt% before 153°C, and an endothermic peak at around 185°C (peak temperature), an exothermic peak at around 188°C, and an endothermic peak at around 231°C were observed. Crystal form D-1 was heated to 90°C, and the heated sample was characterized by XRPD. The results as shown in FIG. 24 showed that the heated sample is a mixture of crystal form B-1 and crystal form D-1.

Crystal form D-2

The crystal form A sample was suspended and stirred in n-propanol for 1 day at room temperature to obtain crystal form D-2 as n-propanol solvate, and the sample was characterized by XRPD (FIG. 7). The TGA/DSC results showed that the sample had a weight loss of about 1.9 wt% before 200°C, and one exothermic peak at around 183°C (peak temperature), one endothermic peak at around 231°C (peak temperature) were observed.

Crystal form D-3

The crystal form A sample was suspended and stirred in 2-butanone for 1 day at room temperature to obtain crystal form D-3 as 2-butanone solvate, which was characterized by XRPD (FIG. 8) and $^1$H-NMR. The $^1$H-NMR results showed that the crystal form D-3 sample contained about 11.04 wt% of 2-butanone solvent. The TGA/DSC results showed that the sample had a weight loss of about 11.07 wt% before 120°C, and an endothermic peak at around 143°C, an exothermic peak at around 153°C, and an endothermic peak at around 230°C were observed.

Similarly, the three crystal forms of crystal form D-1, crystal form D-2 and crystal form D-3 have very similar XRPD patterns and they are considered to have isomorphic structure.

Crystal form E-1

The crystal form A sample was suspended and stirred in 1,4-dioxane at room temperature to obtain crystal form E-1 as 1,4-dioxane solvate, which was characterized by XRPD (FIG. 9) and $^1$H-NMR. The $^1$H-NMR results showed that the crystal form E-1 sample contained about 14.26 wt% of 1,4-dioxane solvent. Crystal form E-1 was heated to 150°C, and the heated sample was characterized by XRPD. The results as shown in FIG. 25 showed that the heated sample is crystal form B-1 of Compound 1.

Crystal form E-2

Crystal form A was suspended and stirred in tetrahydrofuran at room temperature to obtain crystal form E-2 as tetrahydrofuran solvate having an XRPD pattern substantially as shown in FIG. 10.

Crystal form E-3

The crystal form A was suspended and stirred in isopropanol at room temperature to obtain crystal form E-3 as isopropanol solvate, which was characterized by XRPD (FIG. 11) and $^1$H-NMR. The $^1$H-NMR results showed that the crystal form E-3 sample contained about 2.0 wt% of isopropanol solvent. The TGA/DSC results showed that the sample had a weight loss of about 3.10 wt% before 149°C, and an endothermic peak at around 188°C, an exothermic peak at around 196°C, and an endothermic peak at around 230°C were observed.

Similarly, the three crystal forms of crystal form E-1, crystal form E-2 and crystal form E-3 have very similar XRPD patterns and they are considered to have isomorphic structure.

Crystal form C

Crystal form A was added to ethyl acetate at room temperature and stirred for 48 h to obtain crystal form C as anhydrous crystal form having an XRPD pattern substantially as shown in FIG. 21. TGA and DSC results showed that no significant weight loss was observed until the sample was heated to 150°C, and an endothermic peak at around 233°C (peak temperature) was observed.

Crystal form F

Crystal form A was added to dimethyl sulfoxide solvent at room temperature until dissolution and clear, and then the reaction solution was subjected to gas-liquid diffusion under an atmosphere of pure water for 7 days to obtain crystal form F as dimethyl sulfoxide solvate having an XRPD pattern substantially as shown in FIG. 12. Crystal form F was heated to 180°C, and the heated sample was characterized by XRPD. The results as shown in FIG. 26 showed that the crystal form F transformed into crystal form C after heating.

Crystal form G

Crystal form A was added to *N*,*N*-dimethylformamide solvent at room temperature until dissolution and clear, and then the reaction solution was subjected to gas-liquid diffusion under an atmosphere of pure water for 7 days to obtain crystal form G as N,N-dimethylformamide solvate having an XRPD pattern substantially as shown in FIG. 13. Crystal form G was heated to 180°C, and the heated sample was characterized by XRPD. The results as shown in FIG. 27 showed that the crystal form G transformed into crystal form C after heating.

Crystal form H

Crystal form C sample was suspended and stirred in tetrahydrofuran solvent system at room temperature to obtain crystal form H as tetrahydrofuran solvate having an XRPD pattern substantially as shown in FIG. 14. Crystal form H was heated to 150°C, and the heated sample was characterized by XRPD. The results as shown in FIG. 28 showed that the crystal form H transformed into crystal form B-1 after heating.

Crystal form I

The crystal form C sample was added to dimethyl sulfoxide at room temperature until dissolution, followed by the addition of the antisolvent acetonitrile, and the resultant was suspended and stirred at room temperature to obtain crystal form I as acetonitrile solvate having an XRPD pattern substantially as shown in FIG. 15. Crystal form I was heated

to 220°C, and the heated sample was characterized by XRPD. The results as shown in FIG. 29 showed that the crystal form I transformed into crystal form C after heating.

Crystal form J

The crystal form C sample was added to *N*-methyl pyrrolidone at room temperature until dissolution, followed by the addition of the antisolvent isopropanol, and the resultant was suspended and stirred at room temperature to obtain crystal form J as isopropanol solvate having an XRPD pattern substantially as shown in FIG. 16. The sample was heated to 220°C, and the heated sample was characterized by XRPD. The results as shown in FIG. 30 showed that the crystal form J transformed into crystal form C after heating.

Crystal form K

The crystal form C sample was added to the solvent mixture of acetonitrile and trichloromethane at room temperature until dissolution, and the solution was evaporated naturally at room temperature for 5 days to obtain the single crystal crystal form K as trichloromethane solvate having an XRPD pattern substantially as shown in FIG. 17. The analysis results of the single crystal are as follows.

Single crystal detection was carried out using Bruker D8 VENTURE, and the results showed that the crystal belongs to the orthorhombic crystal system, in which the space group is $P2_12_12_1$, the cell parameters are: a = 7.2213(3), b = 14.5964(7), c = 26.9474(12) Å, $\alpha = \beta = \gamma = 90°$, cell volume V = 2840.4(2)Å3, and the number of asymmetric units in the cell Z = 4.

The crystal structure was resolved by the direct method (Shelxs97) to obtain all 39 non-hydrogen atom positions, the least squares method was used to correct the structural parameters and identify the atom types, and the geometrical calculation method and the difference Fourier method were used to obtain all the hydrogen atom positions, with the refined $R_1$ = 0.0995, $wR_2$ = 0.3135 ($w = 1/\sigma|F|^2$), and S = 1.082. The finalized stoichiometric formula was $C_{25}H_{30}N_6O_4 \cdot CHCl_3$, and the calculated molecular weight and the calculated crystal density were 597.92 and 1.398 g/cm$^3$, respectively.

The anhydrous crystal form B-1 and anhydrous crystal form C of Compound 1 not only solved the potential toxicity problem of the solvate of Compound 1, but also greatly improved the stability of the crystal form, and the vast majority of solvate crystal forms were transformed into anhydrous crystal form B-1 or anhydrous crystal form C at a certain temperature.

It was surprisingly found that anhydrous crystal form B-1 and anhydrous crystal form C also have certain advantages in terms of thermodynamic stability, pressure sensitivity and grinding sensitivity. Both anhydrous crystal forms have excellent stability and high melting points, and are free of water molecules, crystalline, and less hydroscopic, which are suitable for use in pharmaceutical formulations.

**Example 5 Preparation of crystal form C of Compound 1**

Compound 1 (0.6 g) was added to 6 mL of a solvent mixture of EA and DMSO (1:1, v/v), and the solution was heated to 50°C for complete dissolution, followed by the dropwise addition of methyl tert-butyl ether (6 mL). After the addition, the heating was stopped, and system was naturally cooled down to room temperature with stirring to precipitate crystals, which were filtered and dried under vacuum at 45°C for 16 h to obtain crystal C as a solid having X-ray powder diffraction pattern substantially as shown in FIG. 21.

Compound 1 (0.6 g) was added to 12 mL of a solvent mixture of EA and MeOH (1:1, v/v), and the solution was heated to 50°C for complete dissolution, followed by the dropwise addition of methyl tert-butyl ether (24 mL). After the addition, the heating was stopped, and system was naturally cooled down to room temperature with stirring to precipitate the crystals, which were filtered and dried under vacuum at 45°C for 16 h to obtain crystal C as a solid having X-ray powder diffraction pattern substantially as shown in FIG. 21.

15 mg of crystal form A of Compound 1 was taken and added to each of the solvents (1 mL to 1.5 mL) in Table 1, suspended and stirred at room temperature for about 3 days to obtain solids. All of the resulted solids were characterized by XRPD as crystal form C. The X-ray powder diffraction pattern of crystal form C is substantially as shown in FIG. 21.

Table 1 List of solvents used in the preparation method for crystal form C

| Solvent | Solid Crystal Form |
|---|---|
| Methanol | Crystal Form C |
| Methyl isobutyl ketone | Crystal Form C |

(continued)

| Solvent | Solid Crystal Form |
|---|---|
| Isopropyl acetate | Crystal Form C |
| Anisole | Crystal Form C |
| N-butyl alcohol | Crystal Form C |
| Methyl tertiary butyl ether | Crystal Form C |
| Methanol/Water(14:1, v/v) | Crystal Form C |
| Methanol/Water (5:1, v/v) | Crystal Form C |
| Methanol/Water (2:1, v/v) | Crystal Form C |

It is worth noting that the crystal form C of Compound 1 of the present invention has a strong selective orientation, and the intensities of several peaks in the XRPD patterns of different samples of the crystal form C vary considerably. Therefore, the intensity data for the 2$\theta$ angle in FIG. 21 and the corresponding peak list are only exemplary and cannot be used for an absolute comparison. When the samples or assay conditions are different, some of the non-strong peaks in their corresponding XRPD patterns in FIG. 21 may show higher intensities and/or some of the strong peaks may be weaker in intensity, but this does not affect the matching of the overall spectra.

**Example 6 Pressure sensitivity assay**

Appropriate amounts of crystal form B-1 and crystal form C of Compound 1 were taken as API, which was subjected to tabletting assay at pressures of 200 kg/cm$^2$, 300 kg/cm$^2$ and 500 kg/cm$^2$, respectively, and the assay results are shown in Table 2.

Table 2 Conditions and results of pressure sensitivity assay

| Sample | Amount of sample (g) | Pressure(kg/cm$^2$) | Tabletting Duration (min) | Result |
|---|---|---|---|---|
| Crystal Form B-1 | 51.10 | 200 | 3 | Crystal Form B-1 |
| | 30.89 | 300 | 3 | |
| | 30.85 | 500 | 3 | |
| Crystal Form C | 30.50 | 200 | 3 | Crystal Form C |
| | 29.51 | 300 | 3 | |
| | 20.39 | 500 | 3 | |

From the assay results, it can be seen that both crystal form B-1 and crystal form C of Compound 1 remained unchanged in terms of crystal form after extrusion under the pressure range of 200 kg/cm$^2$ to 500 kg/cm$^2$, and the XRPD patterns remained consistent with the original patterns, indicating that crystal form B-1 and crystal form C have excellent pressure stability and thus are suitable for industrialized applications.

**Example 7 Grinding sensitivity assay**

Appropriate amounts of crystal form B-1 and crystal form C of Compound 1 were taken into a mortar for manually grinding for 1 min, and the ground samples were characterized by XRPD, the results of which are shown in FIG. 31 and FIG. 32.

As shown in FIG. 31 and FIG. 32, the XRPD patterns of crystal form B-1 and crystal form C of Compound 1 remained consistent with the original patterns after the grinding assay, indicating both crystal form B-1 and crystal form C have excellent grinding stability.

**Example 8 DVS assay**

In order to evaluate the stability of crystal form B-1 and crystal form C under different humidity conditions, crystal form B-1 and crystal form C were subjected to DVS assay between 0% RH to 95% RH at 25°C. The results

showed crystal form B-1 at 80% RH had a mass gain of 1.9% and crystal form C at 80% RH had a mass gain of 0.7%, presuming they have slight hydroscopicity.

**Example 9: Experiments on influencing factor of different crystal forms of Compound 1**

Crystal form M, crystal form B-1 and crystal form C of Compound 1 were taken for stress testing, and the experimental conditions as well as the experimental results of the total impurity contents of the related materials on day 0, day 5 and day 30 are shown in Table 3.

Table 3Stress testing of different crystal forms of Compound 1 - Total impurity content of the related material %

| Sample | Day 0 | High temperature of 40°C | | High temperature of 60°C | | High humidity of 75% | | High humidity of 92.5% | |
|---|---|---|---|---|---|---|---|---|---|
| | | Day 5 | Day 30 | Day 5 | Day 30 | Day 5 | Day 30 | Day 5 | Day 30 |
| Crystal Form M | 0.12 | 0.55 | 0.53 | 0.70 | 0.64 | 0.39 | 0.61 | 0.42 | 0.61 |
| Crystal Form B-1 | 0.24 | 0.26 | 0.34 | 0.38 | 0.43 | 0.25 | 0.29 | 0.23 | 0.24 |
| Crystal Form C | 0.07 | 0.07 | 0.10 | 0.08 | 0.10 | 0.07 | 0.07 | 0.07 | 0.07 |

As can be seen from the above table, in the stress testing, crystal form M of Compound 1 showed a certain increasement in the total impurity content compared with that on day 0 under each condition investigated. Crystal form B-1 and crystal form C showed no significant change in the total impurity content compared with that on day 0 under each condition investigated, and the total impurity contents are all within the limit (the limit for the total impurity content in the quality standard is ≤1.5%), indicating crystal form B-1 and crystal form C of Compound 1 have excellent stability.

**Example 10: Stability test of crystal form C of Compound 1**

The conditions and methods for the stability test of crystal form C of Compound 1 are mainly based on the requirements of "9001 Guidelines for Stability Test of APIs and Pharmaceutical Preparations" of the fourth part general rules of the 2020 edition of the Chinese Pharmacopoeia, as shown in Table 4.

Table 4 Conditions and methods for stability test of crystal form C of Compound 1

| **Item** | | **Placement conditions** | **Investigation duration** | **Investigation items** |
|---|---|---|---|---|
| Influencing factor experiment | High temperature | 40°C | Measurements on day 5, day 10 and day 30, respectively. | Characteristics, weight gain/loss, moisture, related substances, isomers, particle size, content, crystal form |
| | High temperature | 60°C | | |
| | High humidity | 92.5% | | |
| | High humidity | 75% | | |
| Accelerated test | | 40°C±2°C, RH75%±5% | Months 1, 2, 3 and 6 | Characteristics, moisture, related substances, isomer, particle size, content, crystal form |
| Long-term test | | 25°C±2°C, RH60%±5% | Months 3 and 6 | |

Test results: the product was placed uncovered under high temperature conditions or high humidity conditions for 30 days, and there was no significant change in the characteristics, content, crystal form and isomer compared with those on day 0. The product was investigated by the accelerated 6-month and long-term 6-month stability tests, and the

testing indexes showed no significant change compared with those on day 0. It indicates that crystal form C of Compound 1 shows excellent physicochemical stability.

**Example 11: Kinase assay of Compound 1**

PI3Kα, PI3Kβ, PI3Kγ kinases were enzymatically reacted with their substrates ATP and PIP2:3PS, and the amount of the reaction products were determined to reflect the enzymatic activities of PI3Kα, PI3Kβ, PI3Kγ using ADP-Glo reagent and luminescence method (final ATP concentration of 10 μM). The inhibitory activities of Compound 1 of the present invention on PI3Kα, PI3Kβ and PI3Kγ kinases were tested using the above method.

Assay method:

Reagents: basic kinase buffer (pH 7.5); PI3Kα, PI3Kβ, PI3Kγ enzyme solutions; solution of PIP2:3PS and ATP; and ADP-Glo kit (containing 10 mM of $MgCl_2$).

Among them, the buffer components are: 50 mM of Hepes (pH 7.2 to pH 7.5), 3 mM of $MgCl_2$, 1 mM of EGTA, 0.03% of CHAPS, 100 mM of NaCl, and 2 mM of DTT;

Preparation of compound: Compound 1 of the present application was completely dissolved in DMSO and diluted to a specific concentration so that the final concentration of the compound in the incubation system was 1000 nM, 333 nM, 111 nM, 37 nM, 12.4 nM, 4.1 nM, 1.4 nM, 0.46 nM, 0.15 nM and 0.05 nM (PI3Kα); 30,000 nM, 10,000 nM, 3333 nM, 1111 nM, 370 nM, 123 nM, 41 nM, 13.7 nM, 4.6 nM and 1.5 nM (PI3Kβ, PI3Kγ).

Reaction procedure: 1) PI3Kα, PI3Kβ, PI3Kγ protein solutions were added to 384 reaction plates (6008280, PerkinElmer), and the resultants were centrifuged at 1000 rpm for 1 min for later use.

2) The compounds to be tested, negative control DMSO or positive control BYL719 were added to the above 384 reaction plates added with enzymes, and the resultants were centrifuged at 1000 rpm for 1 min, and incubated at 25°C for 15 min.
3) The solution of PIP2:3PS&ATP was added to the above 384 reaction plates, and the resultants were centrifuged at 1000 rpm for 1 min, and incubated at 25°C for 60 min.
4) 5 μL of ADP-Glo reagent (containing 10 mM $MgCl_2$) was transferred to the 384 reaction plates, and the resultants were centrifuged at 1000 rpm for 1 min, and incubated at 25°C for 40 min.
5) 10 μL of Detection reagent was transferred to the 384 reaction plates, and the resultants were centrifuged at 1000 rpm for 1 min, and incubated at 25°C for 40 min.
6) RLU (relative luminescence unit) value were read using Envision multifunctional plate reader. The RLU value was used to characterize the degree of reaction between the enzyme and the substrate, and the $IC_{50}$ value was calculated.
7) Experimental data processing procedure:

Compound inhibition (% inh) = (Negative control RLU - Compound to be tested RLU) / (Negative control RLU - Positive control RLU) * 100%

The following non-linear fitting equation was utilized to obtain the $IC_{50}$ (half inhibitory concentration) of the compound:

$$Y = \text{Minimum Inhibition} + (\text{Maximum Inhibition} - \text{Minimum Inhibition})/(1+10\text{^}((\text{Log}IC_{50}-X)*\text{Slope}));$$

wherein X is the log value of the concentration of the compound to be tested, and Y is the inhibition rate of the compound to be tested (% inh).

The experimental results are shown in Table 5, in which A represents $IC_{50}$ value ≤5 nM; B represents $IC_{50}$ value of 5 nM to 20 nM; C represents $IC_{50}$ value of 300 nM to 600 nM; and D represents $IC_{50}$ value >600 nM.

Table 5

| Examples | $IC_{50}$ of Compound PI3K (nM) | | |
|---|---|---|---|
| | α | β | γ |
| Compound 1 | A | D | D |

As can be seen from Table 5, Compound 1 provided in the present invention has better PI3Kα kinase inhibitory activity and has better PI3Kβ and PI3Kγ kinase subtype selectivity, which can avoid potential side effects caused by multi-target inhibition.

**Example 12: Cell proliferation assay**

Method: CellTiter Glo assay method was used to observe the growth inhibitory effect of Compound 1 of the present application on human tumor cells MCF-7 and HGC-27 cultured in vitro.

Assay method: MCF-7 cells were suspended in DMEM medium to form a cell suspension, and the cell concentration was adjusted to 25,000 cells/mL. HGC-27 cells were suspended in RPMI-1640 medium to form a cell suspension, and the cell concentration was adjusted to 5,000 cells/mL. 100 μL of cell suspension was added to a 96-well plate and placed in a $CO_2$ incubator overnight. Compound 1 was completely dissolved in DMSO and 3-fold gradient dilution was performed to obtain a total of 10 concentrations. The 10 concentrations of the compounds to be tested or the DMSO negative control were transferred to the wells containing 100 μL of medium to give final concentrations of the compounds of 30,000 nM, 10,000 nM, 3,333 nM, 1,111 nM, 370 nM, 123 nM, 41 nM, 13.7 nM, and 4.6 nM, respectively. Incubation was performed at 37°C under 5% $CO_2$ for 96 h for HGC-27 cells and 120 h for MCF-7 cells. Then 100 μL of CellTiter-Glo reagent was added to the above 96-well plate and placed at room temperature for 10 min to stabilize the luminescence signal. The RLU (relative luminescence unit) value was recorded using a VICTOR TM X5 instrument, and then the $IC_{50}$ value was calculated.

Inhibition rate of compound (% inh) = 100% - (RLU of Compound to be tested - RLU of Blank control) / (RLU of Negative control - RLU of Blank control) * 100%

Negative control: DMSO wells;
Blank control: blank medium wells without compound and without cells;
The following non-linear fitting equation was utilized to obtain the $IC_{50}$ (half inhibitory concentration) of the compound:

Y = Minimum inhibition rate + (Maximum inhibition rate - Minimum inhibition rate)/(1+10^((Log$IC_{50}$ - X) * Slope));

wherein X is the log value of the concentration of the compound to be tested, and Y is the inhibition rate of the compound to be tested (% inh).

The experimental data are shown in Tables 6 and 7.

Table 6 $IC_{50}$ assay results of Example compounds on MCF-7 cells

| Examples | $IC_{50}$ of compounds on MCF-7 cells (μM) |
|---|---|
| Control Compound 1 | 0.444 |
| Compound 1 | 0.047 |

Table 7 $IC_{50}$ assay results of Example compounds on HGC-27 cells

| Examples | $IC_{50}$ of compounds on HGC-27 cells (μM) |
|---|---|
| Control Compound 1 | 1.579 |
| Compound 1 | 0.267 |
| Note: The control compound 1 is Example 102 in WO2017001658. | |

As can be seen from the above table, Compound 1 provided in the present application showed better cell proliferation inhibitory activity on cell lines with PI3Kα point mutation and better anti-tumor effect at the same concentration than those of the control compound. Thus, Compound 1 provided in the present application is expected to be a PI3Kα kinase inhibitor with better anti-tumor effect.

**Example 13: Pharmacokinetic assay of crystal form of Compound 1**

Medicament preparation: the control compound, crystal form M of Compound 1 and amorphous form of Compound 2 obtained in Example 1 were weighed and prepared into a 10 mg/mL of solution to be tested in a mixture of 10% of DMSO, 10% of Solutol and 80% of saline; crystal form C of Compound 1 was added into a mixture of 5% of solutol and 95% of 0.5% CMCNa to prepare a 10 mg/mL of solution to be tested.

Assay method: 15 male SD rats with weights: 150 g to 300 g were randomly divided into 5 groups of 3 rats each, and 10 mg/mL of the Example compound was administered by single gavage. The blood was collected through the orbital venous plexus at the designated time points (5 min, 15 min, 30 min, 1 h, 2 h, 4 h, 7 h and 24 h) respectively, and the plasma was separated and stored in a refrigerator at -80°C for spare use. After precipitation of proteins from the above plasma samples by acetonitrile, the supernatant was extracted and mixed with water at 1:1, and 10 μL of which was taken for LC-MS/MS detection. The average value was calculated, and the experimental data were shown in Table 8.

Table 8 Pharmacokinetic assay results of Compound 1 and Compound 2

| Number of compound | Administration by gavage | | |
|---|---|---|---|
| | Dosage (mg/kg) | $C_{max}$ (ng/mL) | AUC (hr*ng/mL) |
| Control Compound 1 | 10 | 1,963 | 7,882 |
| Control Compound 2 | 10 | 362 | 1,089 |
| Compound 2 | 10 | 5,033 | 66,414 |
| Crystal form M of Compound 1 | 10 | 1,597 | 19,832 |
| Crystal form C of Compound 1 | 10 | 3,703 | 36,561 |
| Note: The control compound 2 is Example 101 in WO2017001645. | | | |

## Claims

1. A polymorph of (S)-I-(2-((S)-3-cyclopropyl-5-isopropyl-2,4-dioxoimidazolidin-1-yl)-5,6-dihydrobenzo[*f*]imidazo[1,2-*d*][1,4]oxazepin-9-yl)pyrrolidine-2-carboxamide, wherein the polymorph is crystal form A, and wherein X-ray powder diffraction pattern of the crystal form A has characteristic peaks at 2θ of about 6.2°±0.2°, 7.3°±0.2°, 9.1°±0.2°, 13.6°±0.2° and 14.2°±0.2°.

2. The polymorph according to claim 1, wherein the X-ray powder diffraction pattern has characteristic peaks at 2θ of about 6.2°±0.2°, 7.3°±0.2°, 9.1°±0.2°, 13.6°±0.2°, 14.2°±0.2°, 14.9°±0.2°, 17.1°±0.2° and 17.3°±0.2°.

3. The polymorph according to claim 1 or 2, wherein the X-ray powder diffraction pattern has characteristic peaks at 2θ of about 6.2°±0.2°, 7.3°±0.2°, 9.1°±0.2°, 11.5°±0.2°, 13.6°±0.2°, 14.2°±0.2°, 14.9°±6.2°, 17.1°±0.2°, 17.3°±0.2° and 18.1°±0.2°.

**4.** The polymorph according to any one of claims 1 to 3, wherein the X-ray powder diffraction pattern is substantially as shown in FIG. 1.

**5.** A polymorph of (*S*)-1-(2-((*S*)-3-cyclopropyl-5-isopropyl-2,4-dioxoimidazolidin-l-yl)-5,6-dihydrobenzo[*f*]imidazo[1,2-*d*][1,4]oxazepin-9-yl)pyrrolidine-2-carboxamide, wherein the polymorph is crystal form B-1, and wherein X-ray powder diffraction pattern of the crystal form B-1 has characteristic peaks at 2θ of about 6.1°±0.2°, 7.6°±0.2°, 9.3°±0.2° and 15.4°±0.2°.

**6.** The polymorph according to claim 5, wherein the X-ray powder diffraction pattern has characteristic peaks at 2θ of about 6.1°±0.2°, 7.6°±0.2°, 9.3°±0.2°, 11.6°±0.2° and 15.4°±0.2°.

**7.** The polymorph according to claim 5 or 6, wherein the X-ray powder diffraction pattern has characteristic peaks at 2θ of about 6.1°±0.2°, 7.6°±0.2°, 9.3°±0.2°, 11.6°±0.2°, 14.1°±0.2° and 15.4°±0.2°.

**8.** The polymorph according to any one of claims 5 to 7, wherein the X-ray powder diffraction pattern has characteristic peaks at 2θ of about 6.1°±0.2°, 7.6°±0.2°, 9.3°±0.2°, 11.6°±0.2°, 14.1°±0.2°, 15.4°±0.2°, 16.9°±0.2° and 17.2°±6.2°.

**9.** The polymorph according to any one of claims 5 to 8, wherein the X-ray powder diffraction pattern is substantially as shown in FIG. 18.

**10.** A polymorph of (*S*)-1-(2-((*S*)-3-cyclopropyl-5-isopropyl-2,4-dioxoimidazolidin-l-yl)-5,6-dihydrobenzo[*f*]imidazo[1,2-*d*][1,4]oxazepin-9-yl)pyrrolidine-2-carboxamide, wherein the polymorph is crystal form C, and wherein X-ray powder diffraction pattern of the crystal form C has characteristic peaks at 2θ of about 7.0°±0.2°, 8.4°±6.2° and 10.9°±0.2°.

**11.** The polymorph according to claim 10, wherein the X-ray powder diffraction pattern has characteristic peaks at 2θ of about 5.5°±0.2°, 7.0°±0.2°, 8.4°±0.2°, 10.2°±0.2° and 10.9°±0.2°.

**12.** The polymorph according to claim 10 or 11, wherein the X-ray powder diffraction pattern has characteristic peaks at 2θ of about 5.5°±0.2°, 7.0°±0.2°, 8.4°±0.2°, 9.8°±0.2°, 10.2°±0.2° and 10.9°±0.2°.

**13.** The polymorph according to any one of claims 10 to 12, wherein the X-ray powder diffraction pattern has characteristic peaks at 20 of about 5.5°±0.2°, 7.0°±0.2°, 8.4°±0.2°, 9.8°±0.2°, 10.2°±0.2°, 10.9°±0.2°, 13.5°±0.2° and 13.9°±0.2°.

**14.** The polymorph according to any one of claims 10 to 13, wherein the X-ray powder diffraction pattern is substantially as shown in FIG. 21.

**15.** A pharmaceutical composition, comprising a therapeutically effective amount of a polymorph of (*S*)-1-(2-((*S*)-3-cyclopropyl-5-isopropyl-2,4-dioxoimidazolidin-1-yl)-5,6-dihydrobenzo[*f*]imidazo[1,2-*d*][1,4]oxazepin-9-yl)pyrrolidine-2-carboxamide and a pharmaceutically acceptable excipient, adjuvant and/or carrier.

**16.** The pharmaceutical composition according to claim 15, wherein the polymorph is one or more of the polymorph according to any one of claims 1 to 14.

**17.** Use of the polymorph according to any one of claims 1 to 14 or the pharmaceutical composition according to any one of claims 15 to 16 in the preparation of a medicament for treating, preventing, delaying or stopping an onset or progression of cancer or cancer metastasis.

**18.** Use of the polymorph according to any one of claims 1 to 14 or the pharmaceutical composition according to any one of claims 15 to 16 in the preparation of a medicament for treating a PI3K-mediated disease.

**19.** The use according to claim 18, wherein the PI3K is PI3Kα.

**20.** The use according to claim 18 or 19, wherein the disease is cancer.

**21.** The use according to claim 17 or 20, wherein the cancer is selected from the group consisting of sarcoma, prostate cancer, breast cancer, pancreatic cancer, lung cancer, gastrointestinal cancer, colorectal cancer, thyroid cancer,

liver cancer, head and neck cancer, adrenal cancer, glioma, endometrial carcinoma, melanoma, kidney cancer, bladder cancer, uterine cancer, vaginal cancer, ovarian cancer, multiple myeloma, esophageal cancer, leukemia, brain cancer, oropharyngeal carcinoma, laryngocarcinoma, lymphoma, basal cell carcinoma, polycythemia vera and essential thrombocythemia.

**22.** A method for treating cancer, comprising administering to a subject in need a therapeutically effective amount of the polymorph according to any one of claims 1 to 14 or the pharmaceutical composition according to any one of claims 15 to 16, wherein the cancer is selected from the group consisting of sarcoma, prostate cancer, breast cancer, pancreatic cancer, lung cancer, gastrointestinal cancer, colorectal cancer, thyroid cancer, liver cancer, head and neck cancer, adrenal cancer, glioma, endometrial carcinoma, melanoma, kidney cancer, bladder cancer, uterine cancer, vaginal cancer, ovarian cancer, multiple myeloma, esophageal cancer, leukemia, brain cancer, oropharyngeal carcinoma, laryngocarcinoma, lymphoma, basal cell carcinoma, polycythemia vera and essential thrombocythemia.

**23.** A method for preparing the polymorph according to any one of claims 10 to 14, wherein the polymorph is crystal form C of Compound 1, the Compound 1 is (*S*)-1-(2-((*S*)-3-cyclopropyl-5-isopropyl-2,4-dioxoimidazolidin-1-yl)-5,6-dihydrobenzo[*f*]imidazo[1,2-*d*][1,4]oxazepin-9-yl)pyrrolidine-2-carboxamide, and the method comprises the following steps:

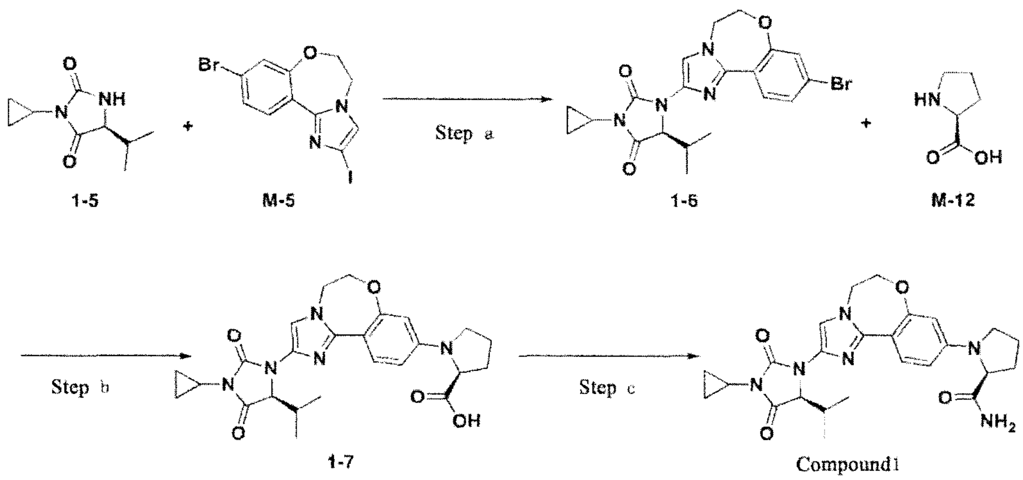


1-5 M-5 1-6 M-12

1-7 Compound1

a. obtaining Compound 1-6 by coupling reaction of Compound 1-5 and Compound M-5 in the presence of a catalyst and a catalyst ligand under a basic condition;
b. obtaining Compound 1-7 by coupling reaction of Compound 1-6 and Compound M-12 in the presence of a catalyst under a basic condition;
c. forming Compound 1 by condensation reaction of Compound 1-7 and $NH_3$ in the presence of a condensing agent under a basic condition; and
g. adding Compound 1 to a good solvent and heating until dissolution, then adding a poor solvent dropwise, precipitating a solid, filtering and drying the solid to give the crystal form C of Compound 1.

**24.** The method according to claim 23, wherein the good solvent is selected from the group consisting of ethyl acetate, a solvent mixture of ethyl acetate and dimethyl sulfoxide, and a solvent mixture of ethyl acetate and methanol; and the poor solvent is selected from the group consisting of n-heptane, methyl tert-butyl ether, isopropyl ether, ethyl ether and a combination thereof.

**25.** A method for preparing the polymorph according to any one of claims 10 to 14, wherein the polymorph is crystal form C of Compound 1, the Compound 1 is (*S*)-1-(2-((*S*)-3-cyclopropyl-5-isopropyl-2,4-dioxo imidazo lid in-1-yl)-5,6-dihydrobenzo[*f*]imidazo [1,2-*d*][1,4]oxazepin-9-yl)pyrro lidine-2-carboxamide, and the method comprises the following steps: adding crystal form A of Compound 1 to a solvent selected from the group consisting of ethyl acetate, methanol, methanol/water mixture, n-butanol, methyl isobutyl ketone, isopropyl acetate, methyl tert-butyl ether and a combination thereof, and suspending and stirring the resultant at room temperature for 1 day to 5 days.

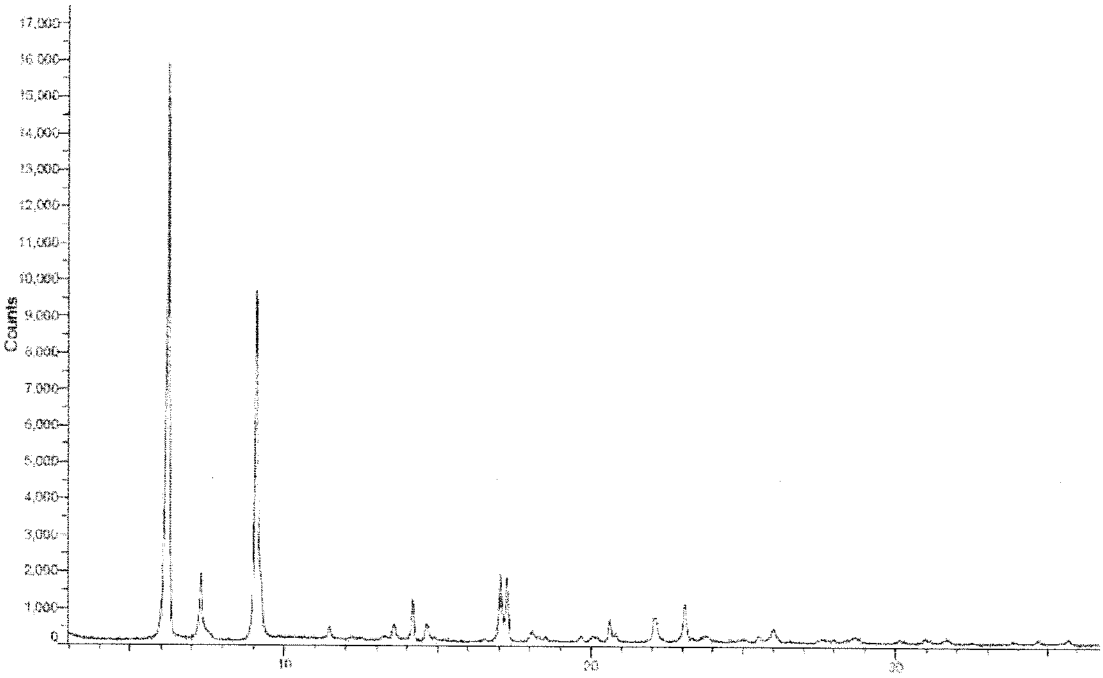
17,000
16,000
15,000
14,000
13,000
12,000
11,000
10,000
9,000
8,000
7,000
6,000
5,000
4,000
3,000
2,000
1,000
0
Counts
10
20
30

FIG. 1

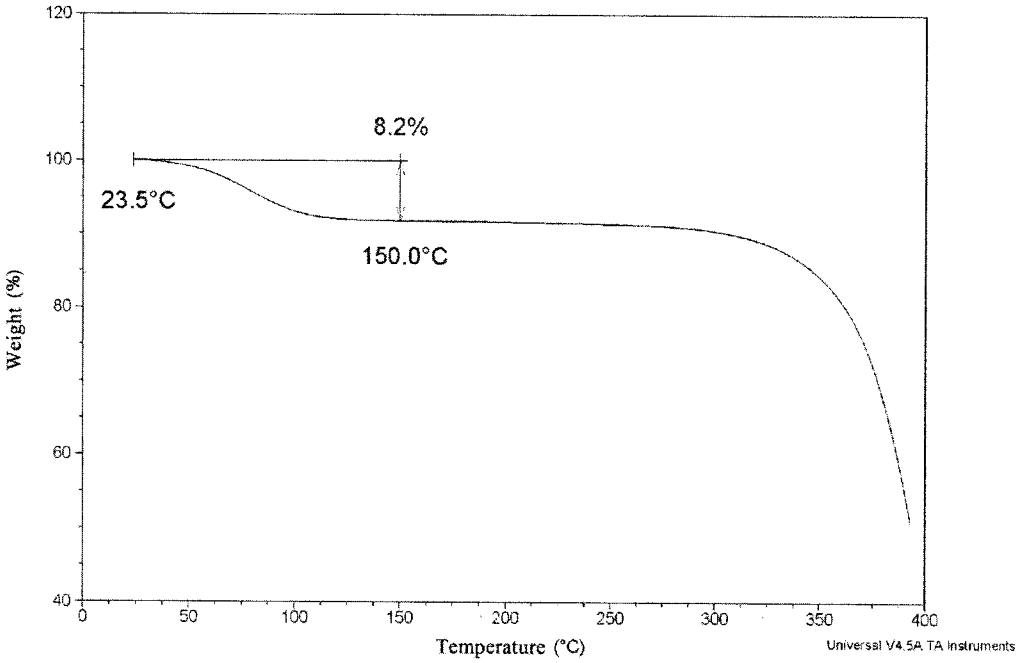
120
100
80
60
40
Weight (%)
8.2%
23.5°C
150.0°C
0
50
100
150
200
250
300
350
400
Temperature (°C)
Universal V4.5A TA Instruments

FIG. 2

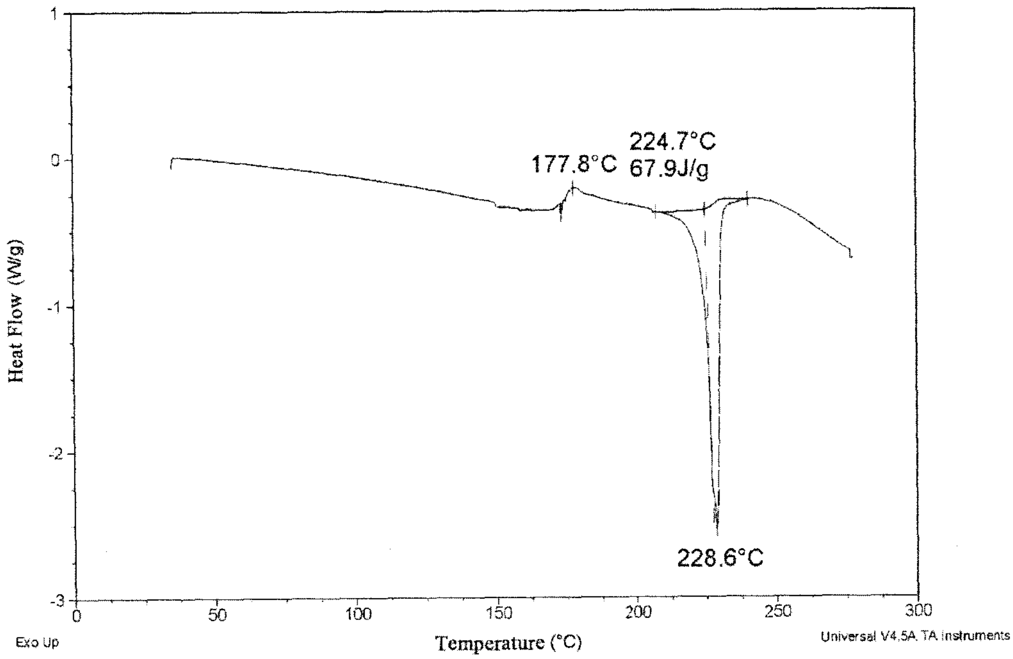
1
0
-1
-2
-3
Heat Flow (W/g)
177.8°C
224.7°C
67.9J/g
228.6°C
0
50
100
150
200
250
300
Exo Up
Temperature (°C)
Universal V4.5A TA Instruments

FIG. 3

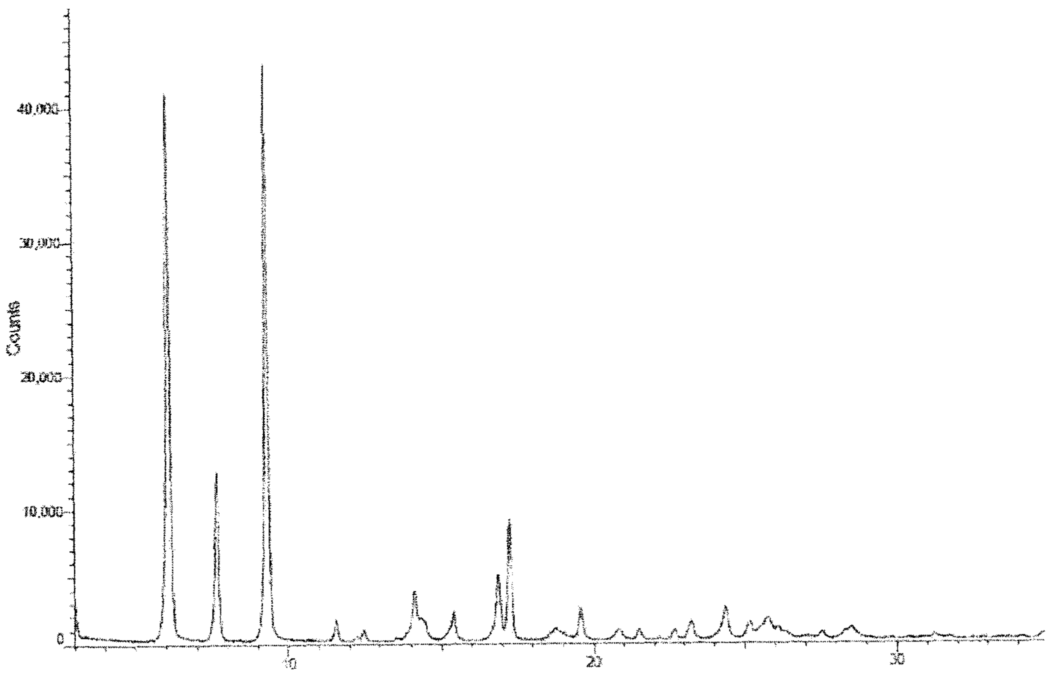
40,000
30,000
20,000
10,000
0
Counts
10
20
30

FIG. 4

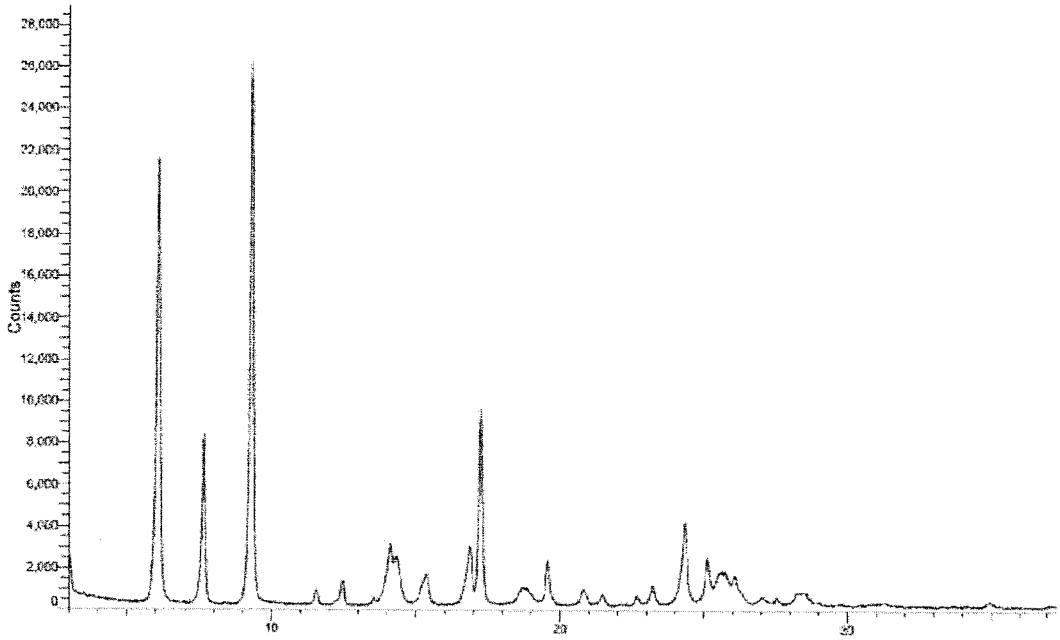

28,000
26,000
24,000
22,000
20,000
18,000
16,000
14,000
12,000
10,000
8,000
6,000
4,000
2,000
0
Counts
10
20
30


FIG. 5

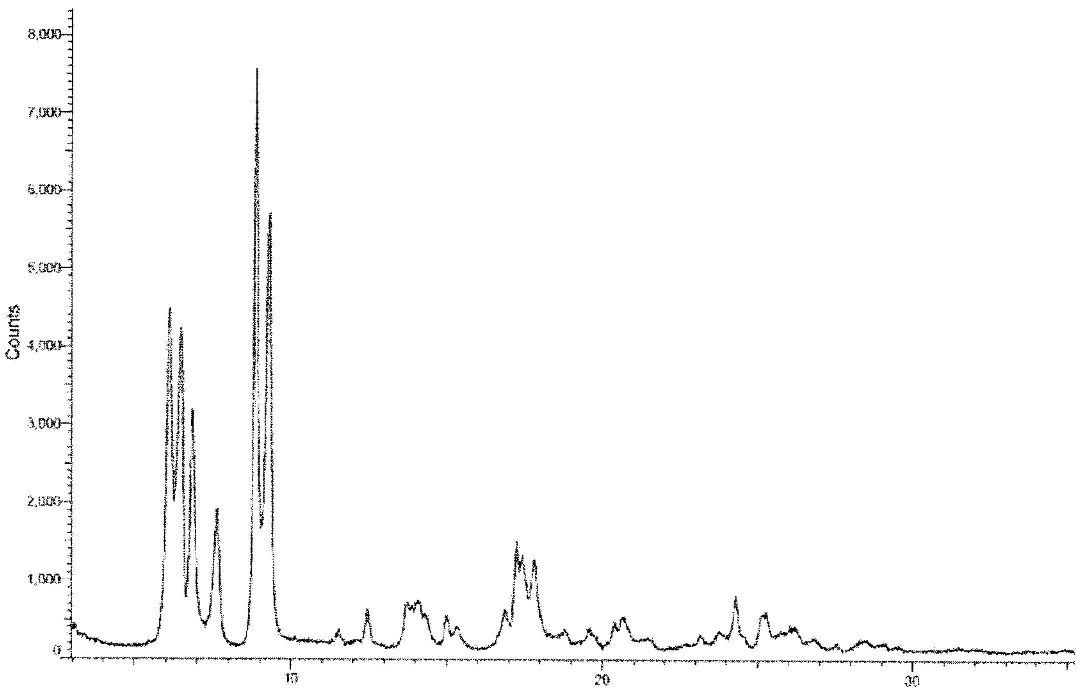

8,000
7,000
6,000
5,000
4,000
3,000
2,000
1,000
0
Counts
10
20
30


FIG. 6

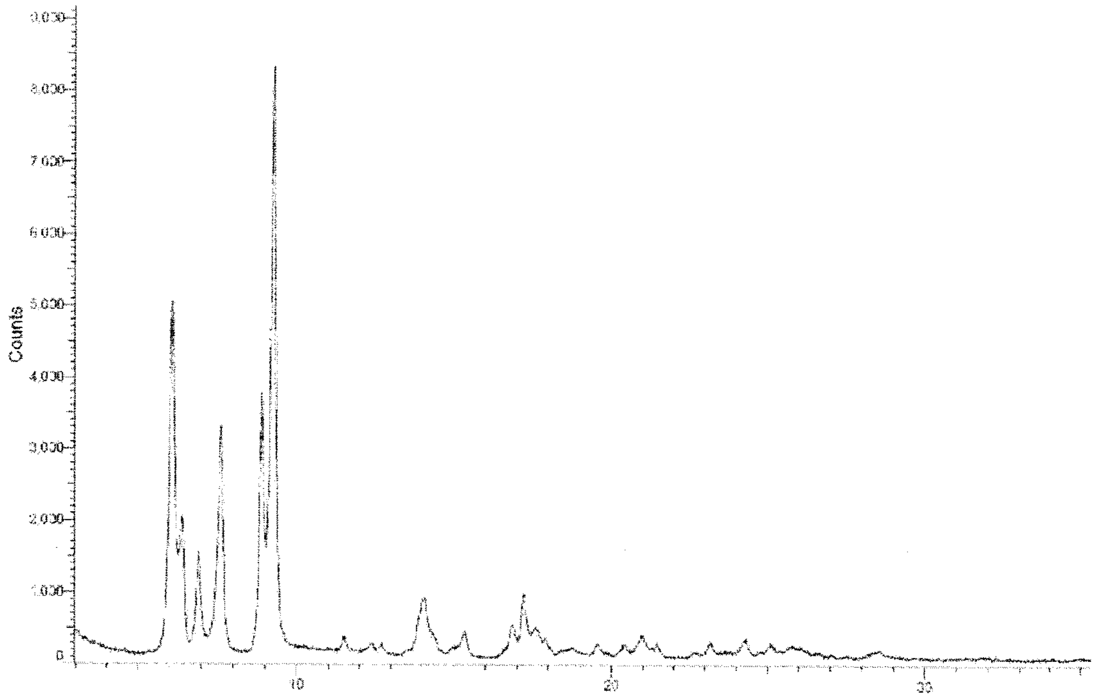
Counts
0
1,000
2,000
3,000
4,000
5,000
6,000
7,000
8,000
10
20
30

FIG. 7

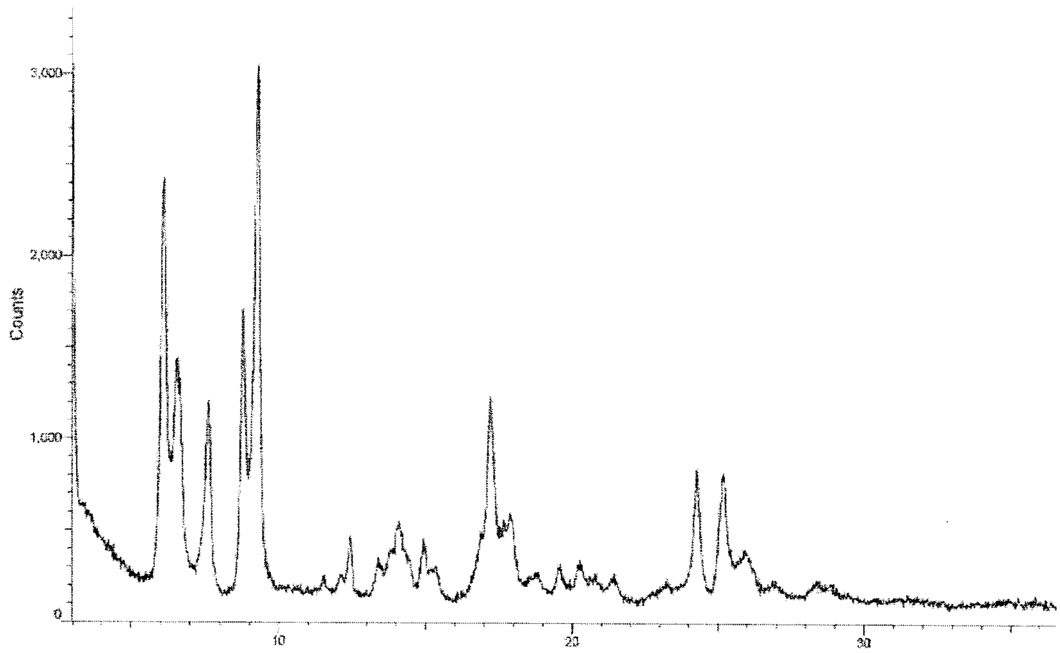
Counts
0
1,000
2,000
3,000
10
20
30

FIG. 8

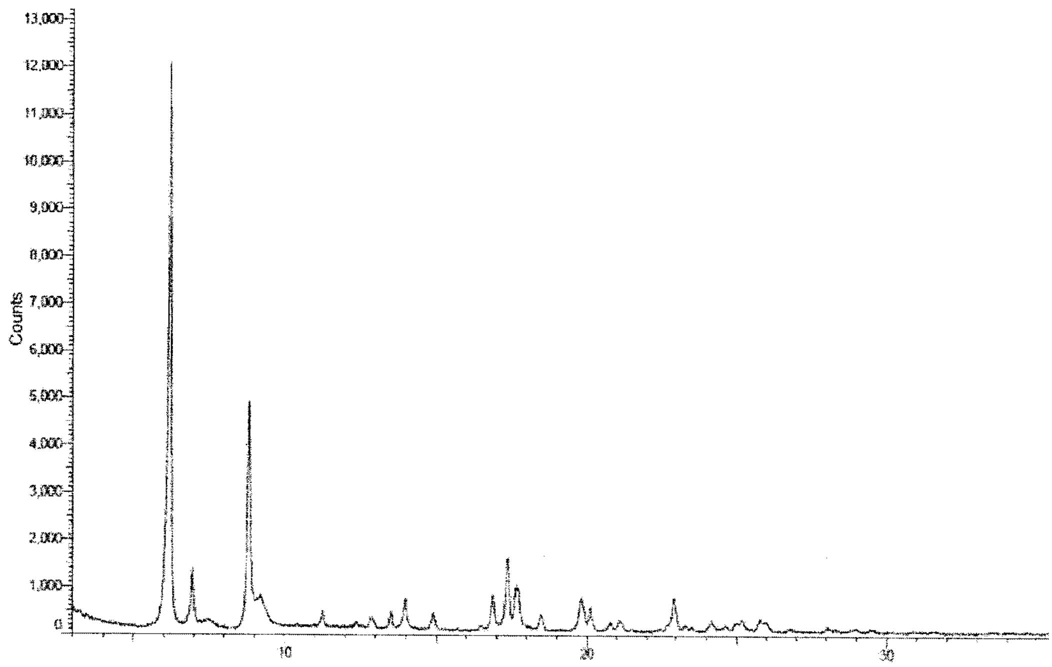
13,000
12,000
11,000
10,000
9,000
8,000
7,000
6,000
5,000
4,000
3,000
2,000
1,000
0
Counts
10
20
30

FIG. 9

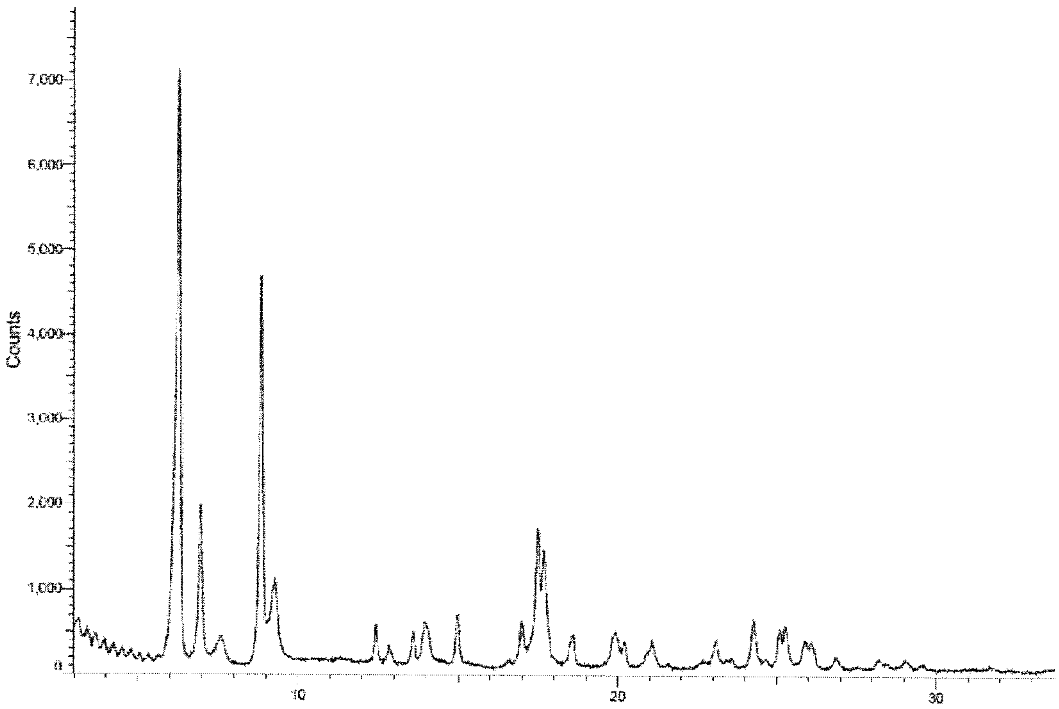
7,000
6,000
5,000
4,000
3,000
2,000
1,000
0
Counts
10
20
30

FIG. 10

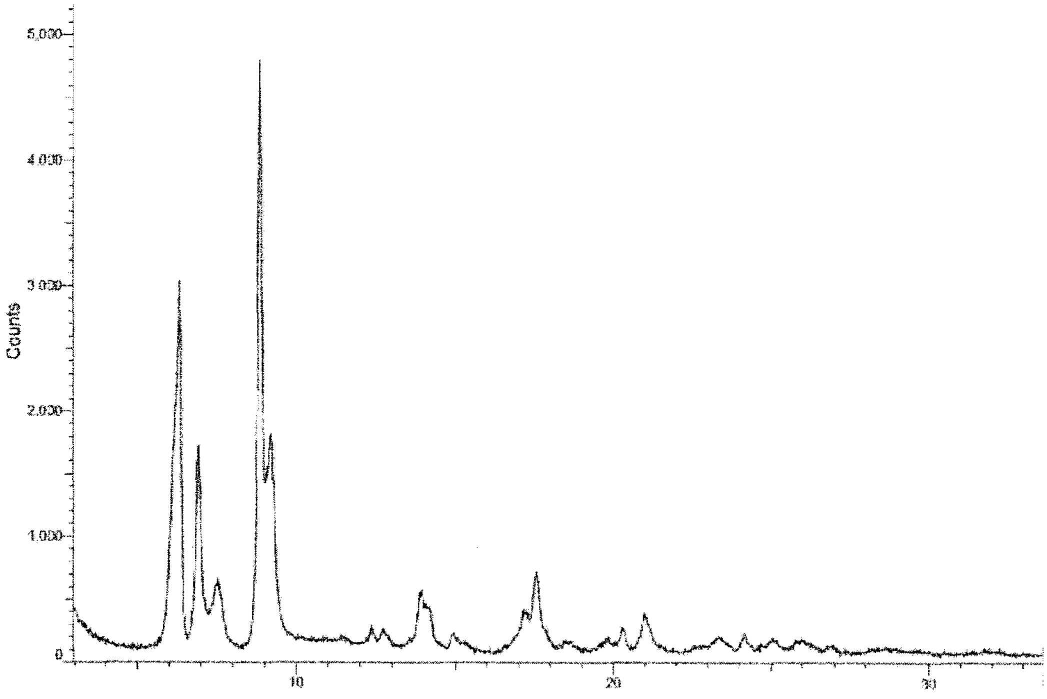
5,000
4,000
3,000
2,000
1,000
0
Counts
10
20
30

FIG. 11

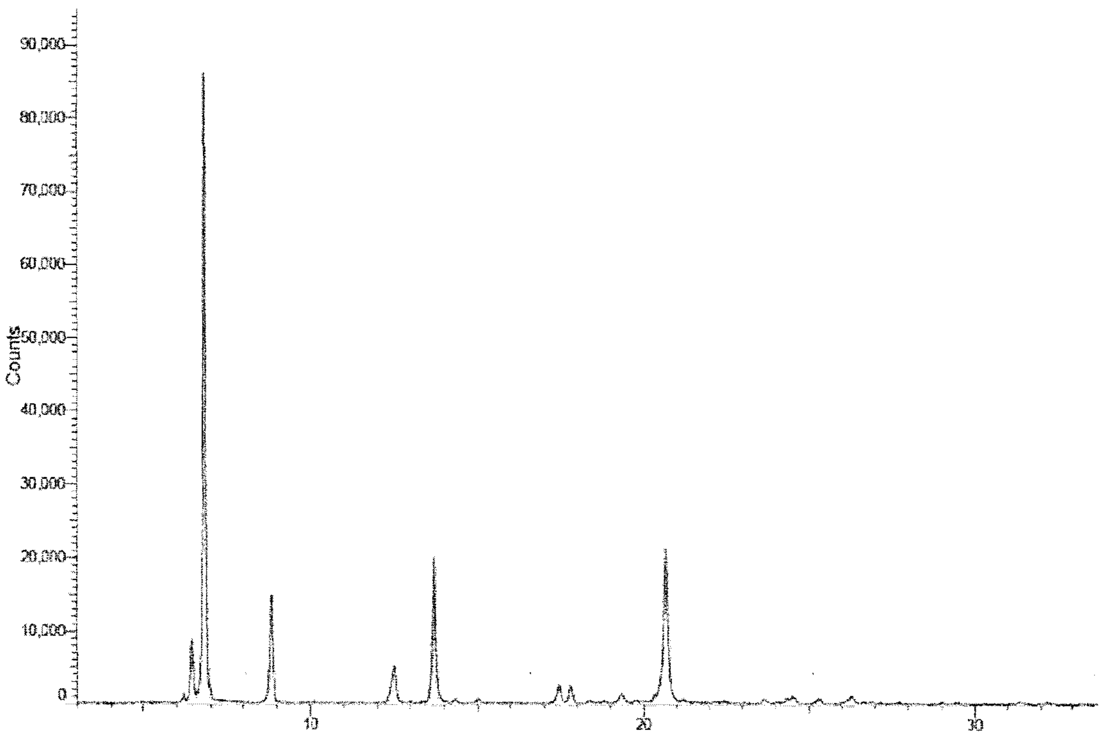
90,000
80,000
70,000
60,000
50,000
40,000
30,000
20,000
10,000
0
Counts
10
20
30

FIG. 12

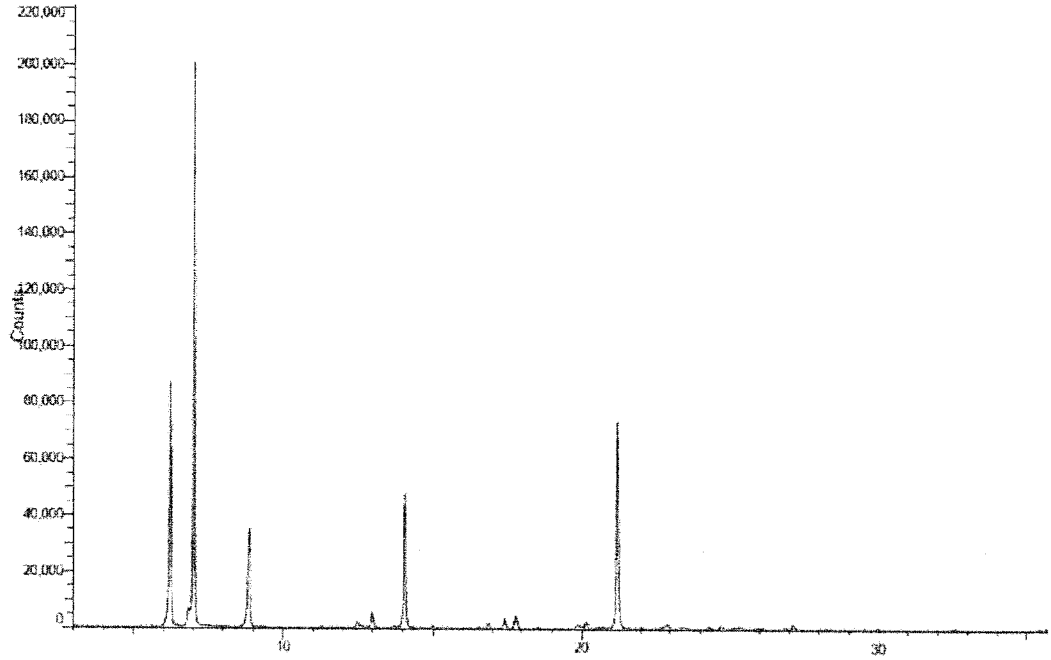
220,000
200,000
180,000
160,000
140,000
120,000
100,000
80,000
60,000
40,000
20,000
0
Counts
10
20
30

FIG. 13

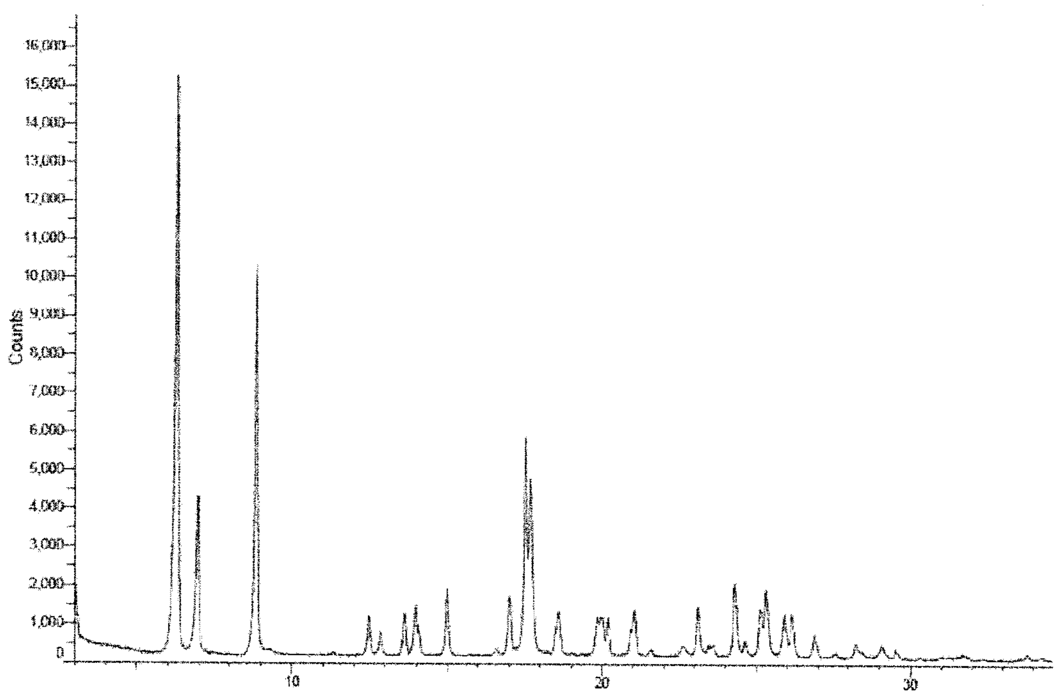
16,000
15,000
14,000
13,000
12,000
11,000
10,000
9,000
8,000
7,000
6,000
5,000
4,000
3,000
2,000
1,000
0
Counts
10
20
30

FIG. 14

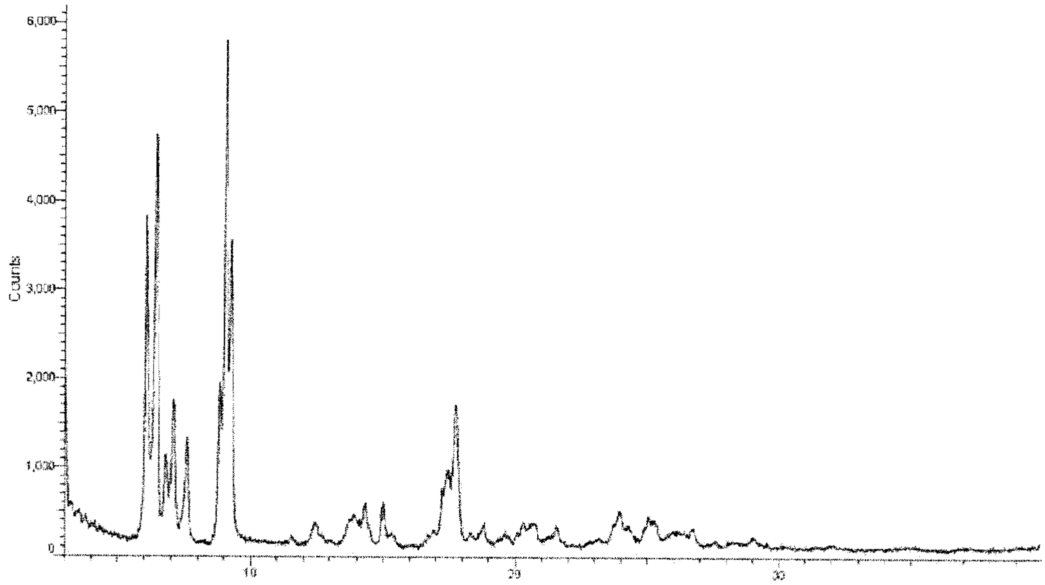
6,000
5,000
4,000
3,000
2,000
1,000
0
Counts
10
20
30

FIG. 15

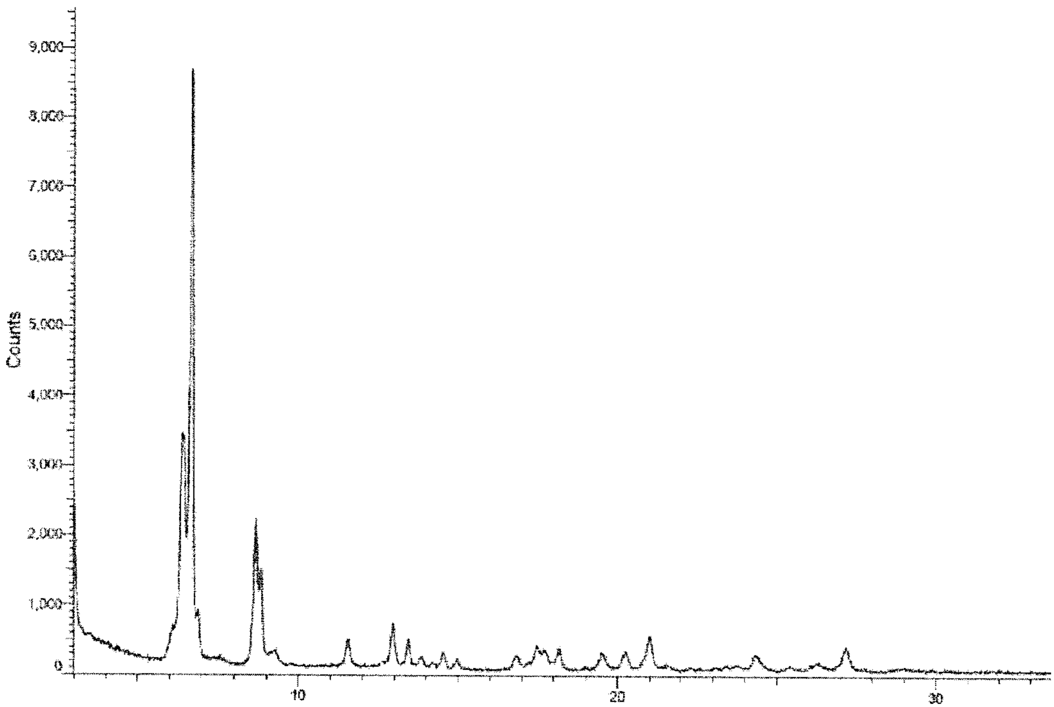
9,000
8,000
7,000
6,000
5,000
4,000
3,000
2,000
1,000
0
Counts
10
20
30

FIG. 16

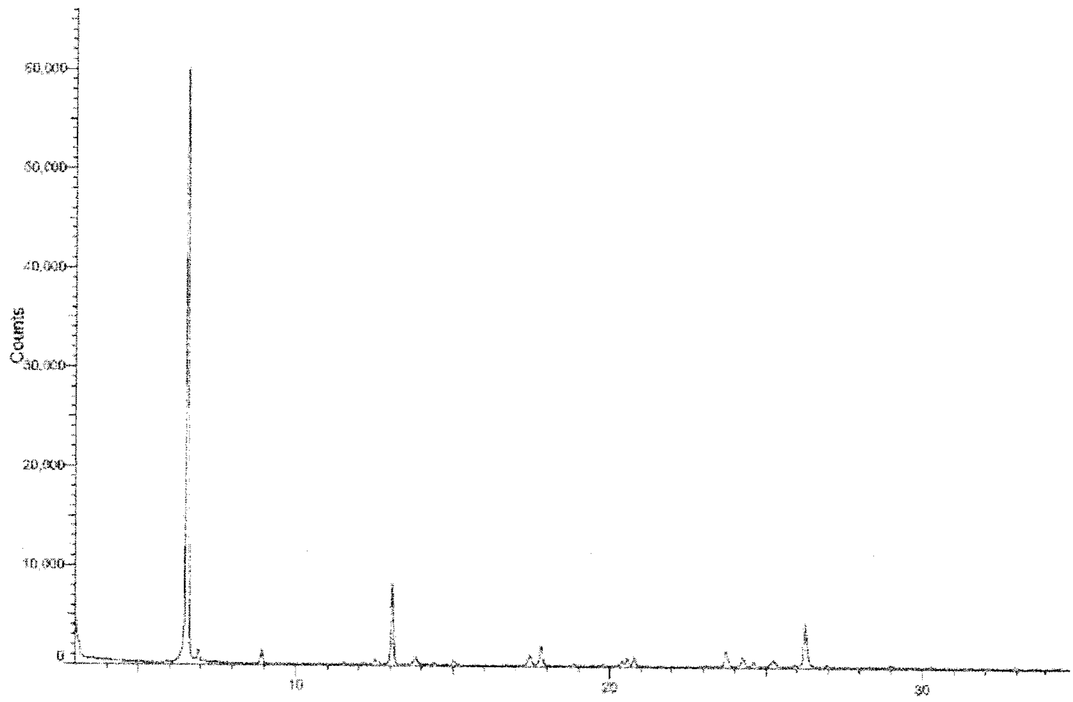
60,000
50,000
40,000
30,000
20,000
10,000
0
Counts
10
20
30

FIG. 17

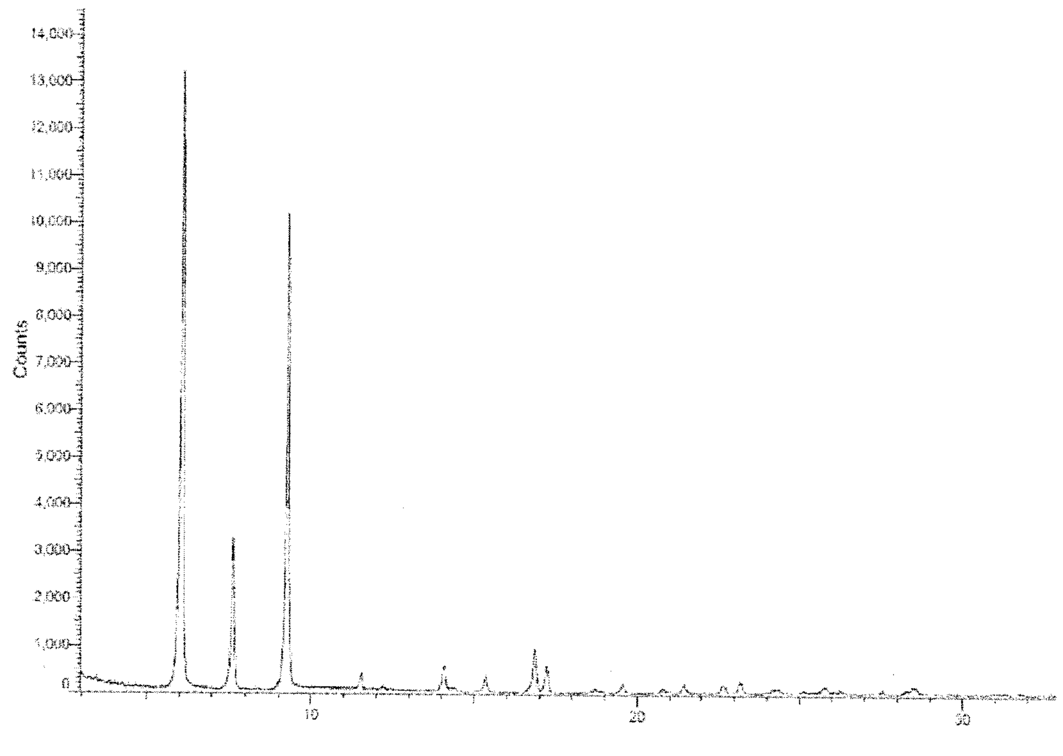
14,000
13,000
12,000
11,000
10,000
9,000
8,000
7,000
6,000
5,000
4,000
3,000
2,000
1,000
0
Counts
10
20
30

FIG. 18

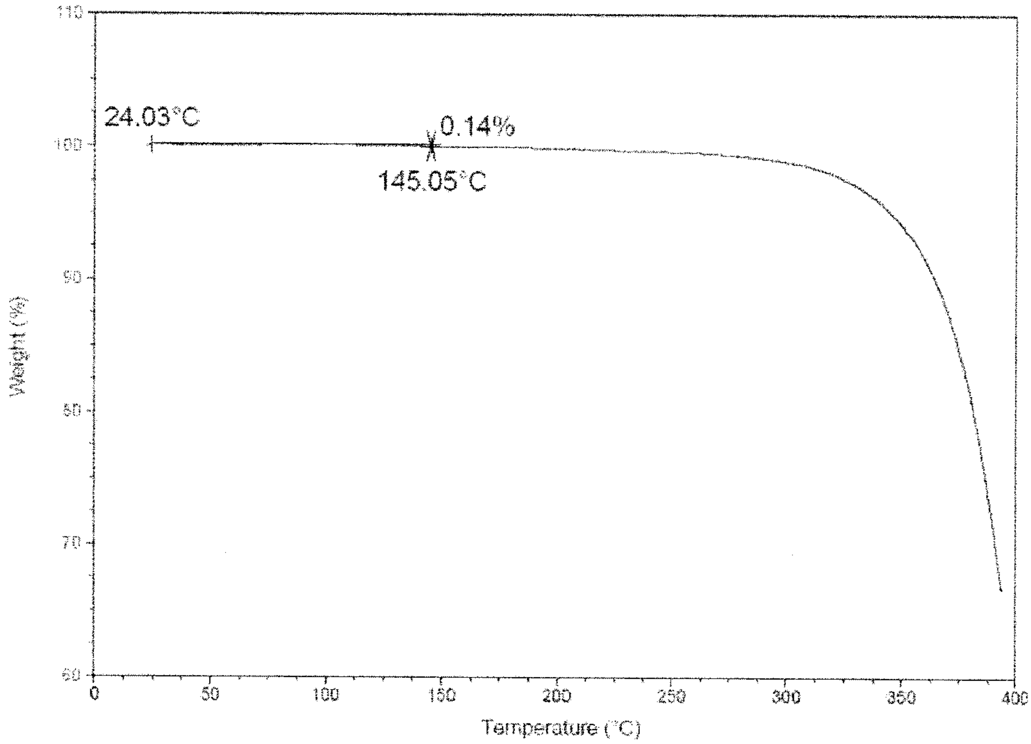
24.03°C
0.14%
145.05°C
Weight (%)
Temperature (°C)

FIG. 19

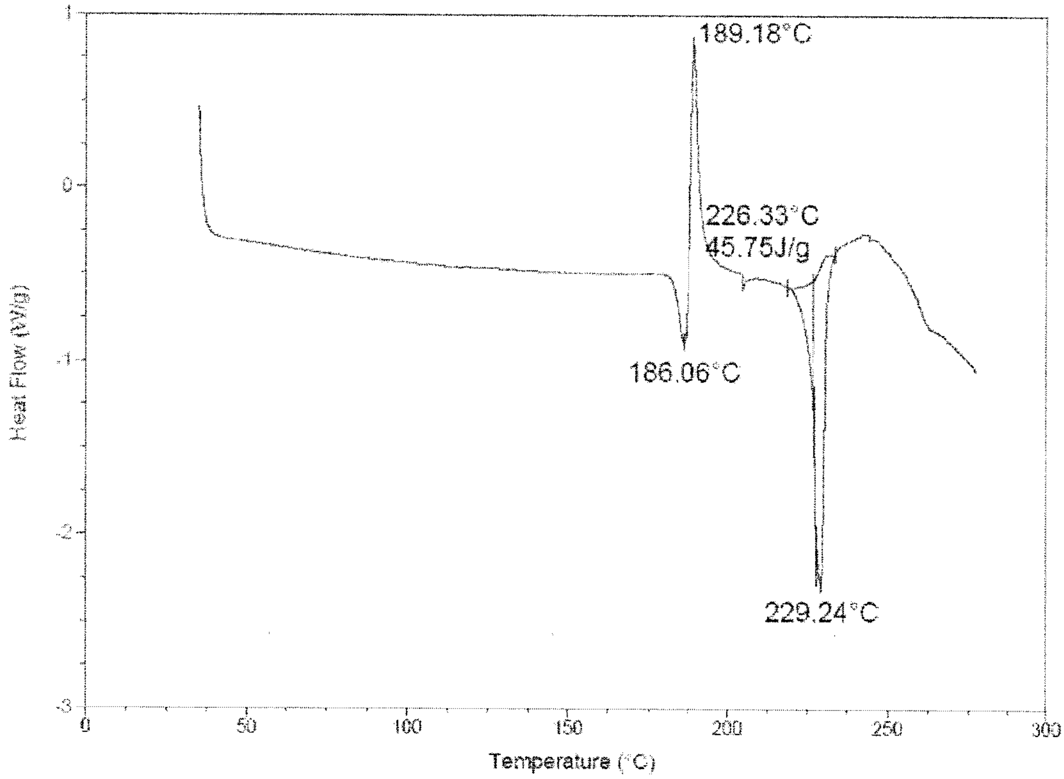
189.18°C
226.33°C
45.75J/g
186.06°C
229.24°C
Heat Flow (W/g)
Temperature (°C)

FIG. 20

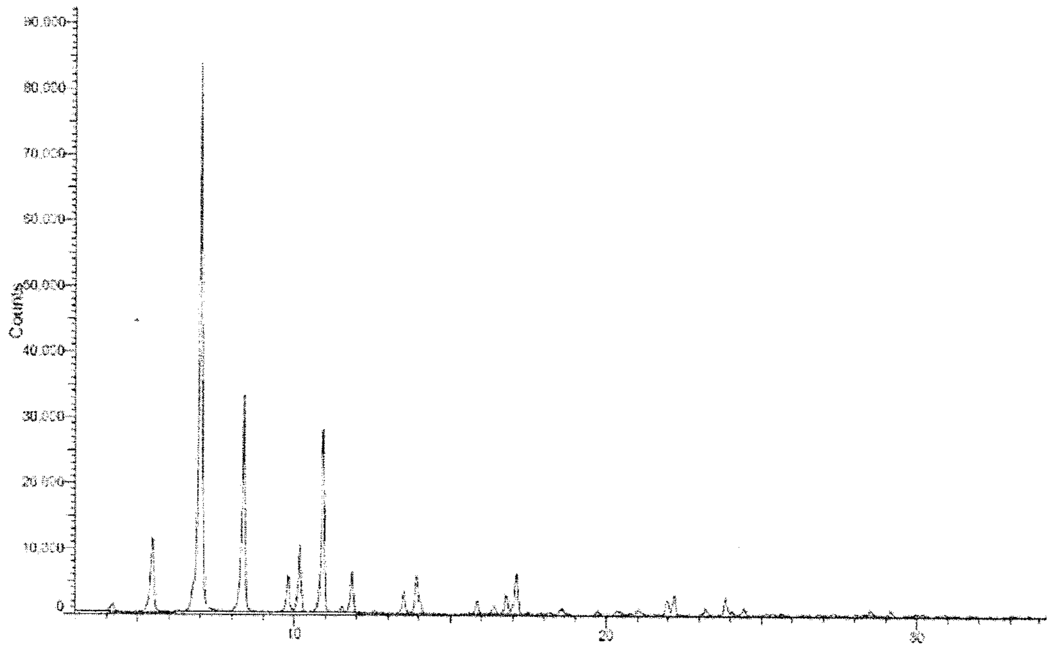
90,000
80,000
70,000
60,000
50,000
40,000
30,000
20,000
10,000
0
Counts
10
20
30

FIG. 21

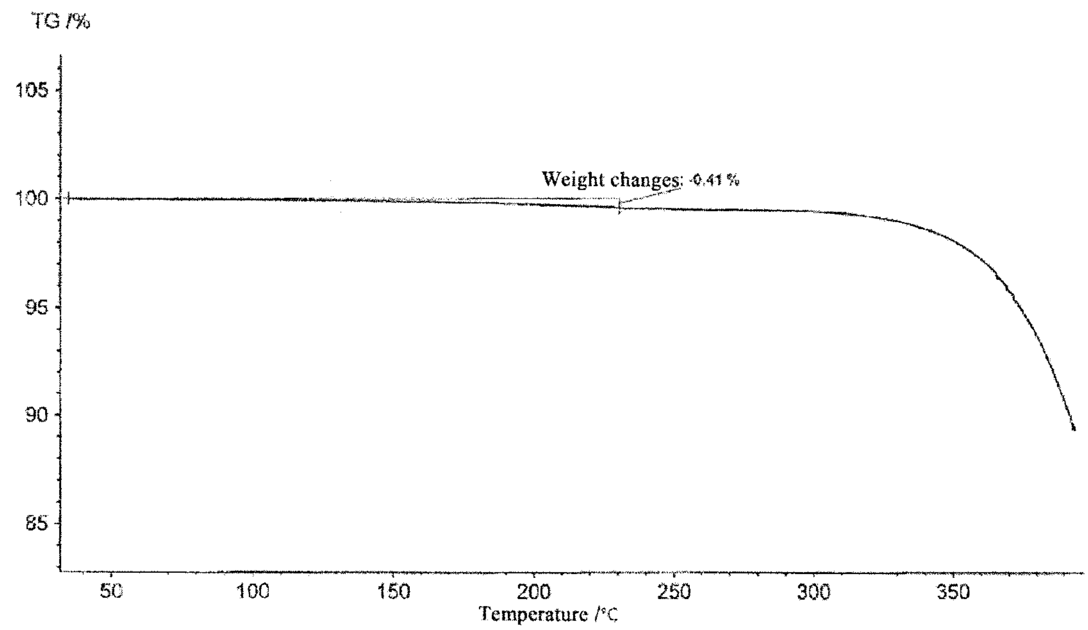
TG /%
105
100
95
90
85
Weight changes: -0.41 %
50
100
150
200
250
300
350
Temperature /°C

FIG. 22

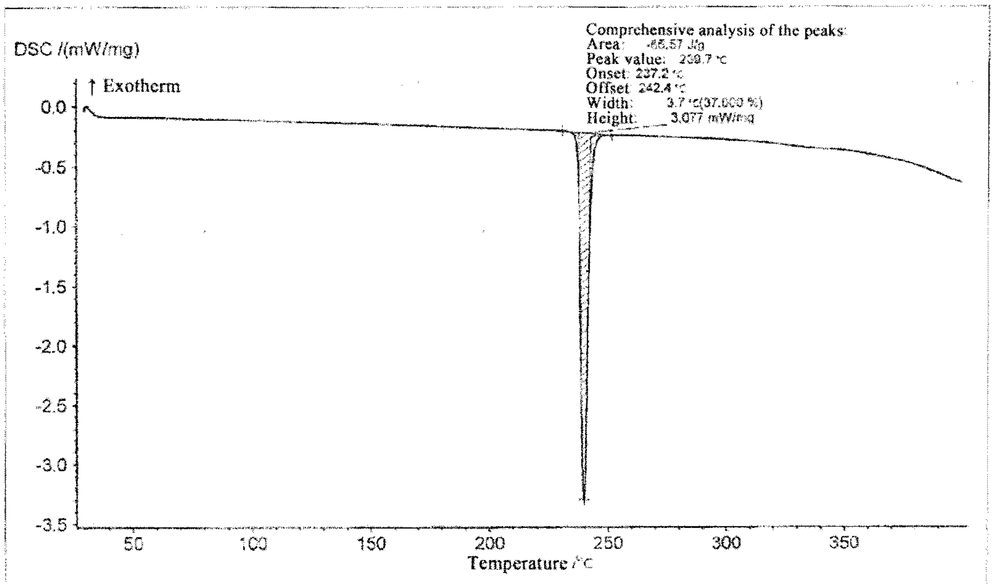


FIG. 23

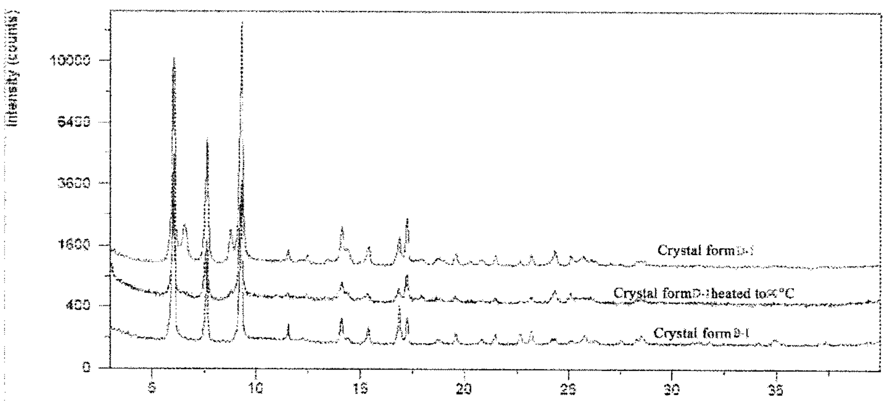


FIG. 24

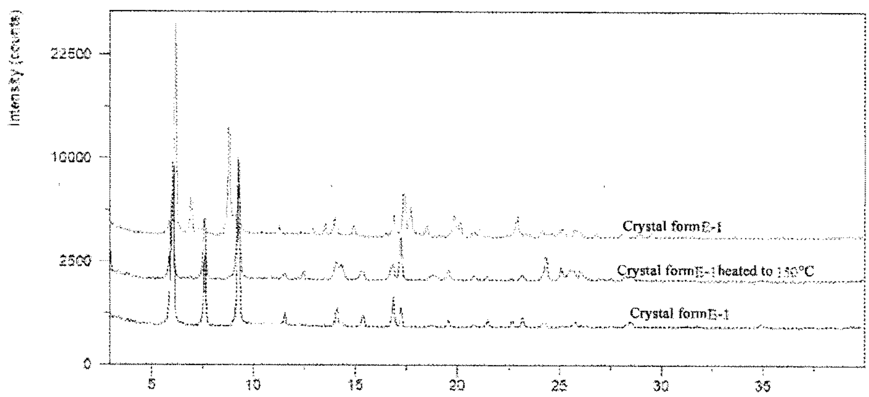


FIG. 25

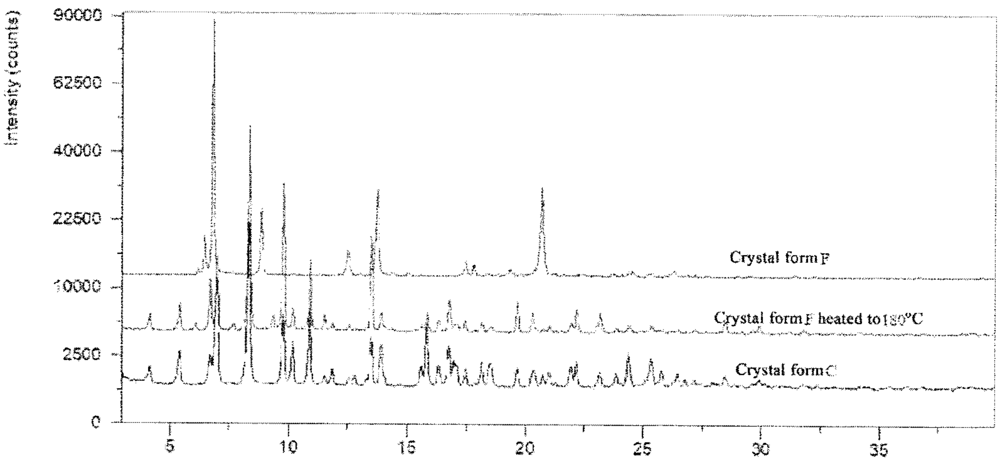


FIG. 26

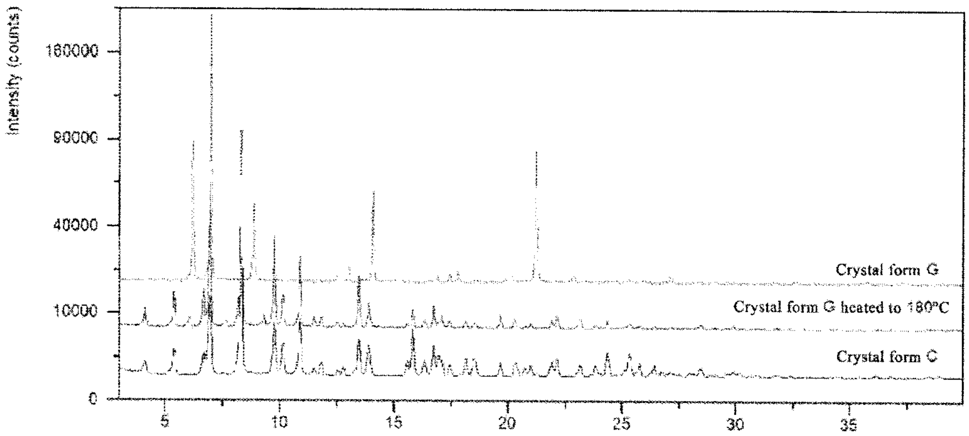


FIG. 27

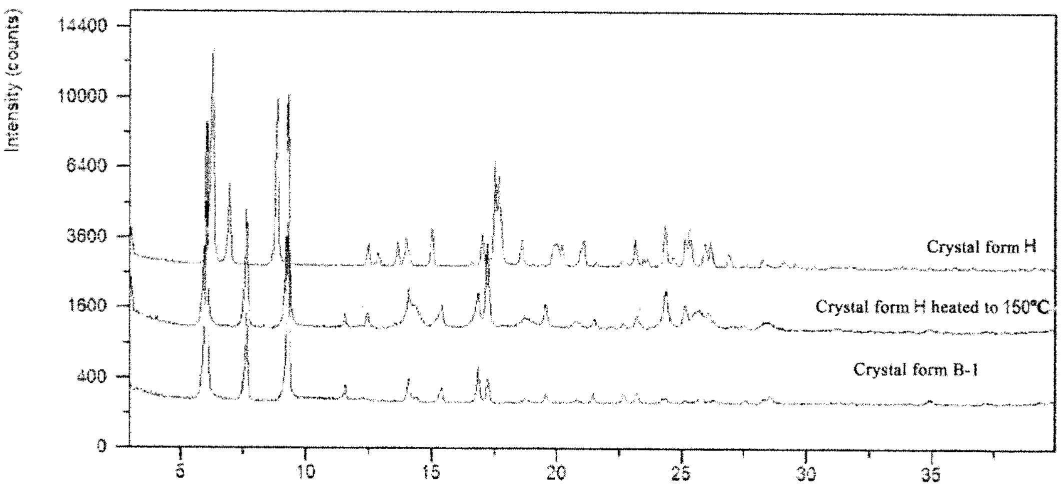


FIG. 28

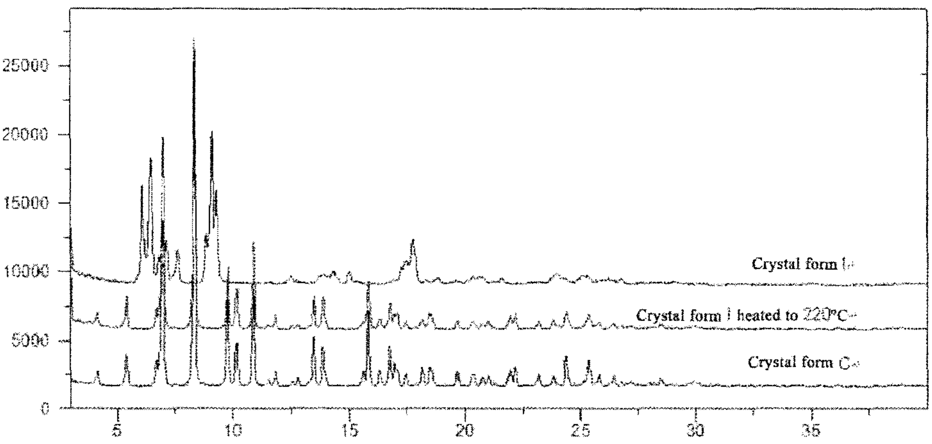


FIG. 29

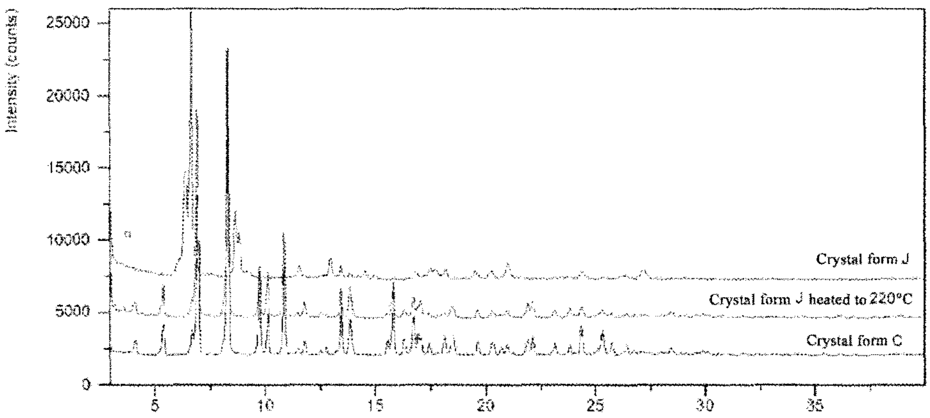


FIG. 30

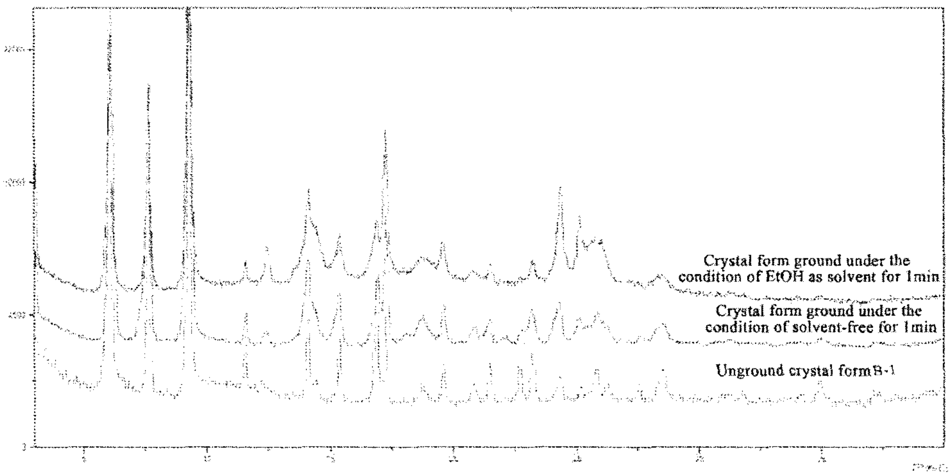


FIG. 31

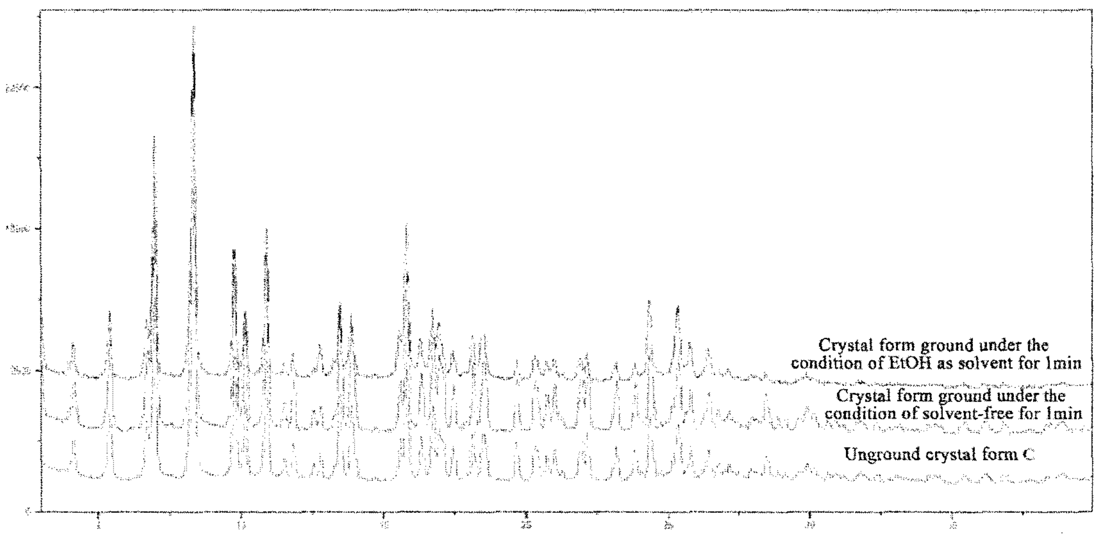
Crystal form ground under the condition of EtOH as solvent for 1min
Crystal form ground under the condition of solvent-free for 1min
Unground crystal form C

FIG. 32

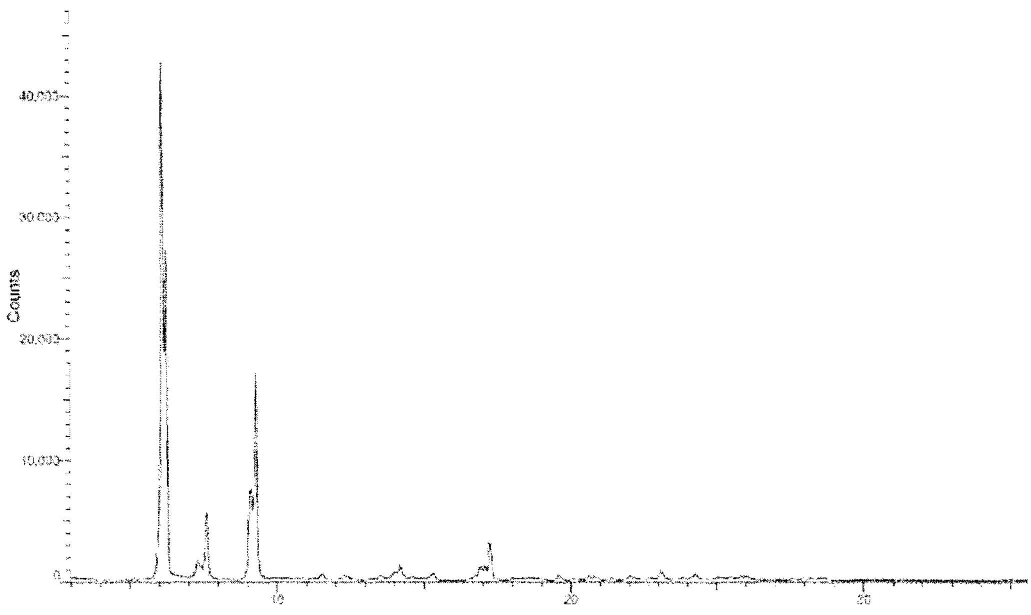
Counts
40,000
30,000
20,000
10,000
0
10
20
30

FIG. 33

## INTERNATIONAL SEARCH REPORT

International application No. **PCT/CN2022/092465**

**A. CLASSIFICATION OF SUBJECT MATTER**

C07D 498/04(2006.01)i; A61K 31/4188(2006.01)i; A61P 35/00(2006.01)i

According to International Patent Classification (IPC) or to both national classification and IPC

**B. FIELDS SEARCHED**

Minimum documentation searched (classification system followed by classification symbols)

C07D; A61K; A61P

Documentation searched other than minimum documentation to the extent that such documents are included in the fields searched

Electronic data base consulted during the international search (name of data base and, where practicable, search terms used)

WPI, EPODOC, STN(REGISTRY, CAPLUS), CNTXT: 贝达药业, 咪唑烷酮, 氧氮杂卓, PI3K, 肿瘤, 癌症, imidazolidin+, benzoxazepin, tumo?r, cancer, 2641606-93-9, 2641606-63-3, 2641606-92-8

**C. DOCUMENTS CONSIDERED TO BE RELEVANT**

| Category* | Citation of document, with indication, where appropriate, of the relevant passages | Relevant to claim No. |
|---|---|---|
| PX | WO 2021088839 A1 (BETTA PHARMACEUTICALS CO., LTD.) 14 May 2021 (2021-05-14) description, pp. 23-26, embodiment 2, and p. 35, compound 33, and claims 24-41 | 1-25 |
| X | CN 102762576 A (F. HOFFMANN-LA ROCHE AG) 31 October 2012 (2012-10-31) claims 8-11, and description, paragraphs [0026]-[0033], [0107]-[0108], and [0168]-[0170], and pp. 77, 94, and 108 | 1-22, 25 |
| A | CN 103562210 A (F. HOFFMANN-LA ROCHE AG) 05 February 2014 (2014-02-05) claims 1, 12, and 19-27, and description, p. 80 | 1-25 |
| A | CN 107995911 A (F. HOFFMANN-LA ROCHE AG) 04 May 2018 (2018-05-04) claims 7-11, and description, p. 16 | 1-25 |

☐ Further documents are listed in the continuation of Box C. ☑ See patent family annex.

* Special categories of cited documents:

"A" document defining the general state of the art which is not considered to be of particular relevance

"E" earlier application or patent but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
|---|---|
| **27 July 2022** | **11 August 2022** |
| Name and mailing address of the ISA/CN<br>**China National Intellectual Property Administration (ISA/CN)**<br>**No. 6, Xitucheng Road, Jimenqiao, Haidian District, Beijing 100088, China** | Authorized officer |
| Facsimile No. **(86-10)62019451** | Telephone No. |

Form PCT/ISA/210 (second sheet) (January 2015)

**INTERNATIONAL SEARCH REPORT**

International application No.
**PCT/CN2022/092465**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **22**
because they relate to subject matter not required to be searched by this Authority, namely:

[1] The subject matter of claim 22 belongs to a method for treating a disease of a human body as defined in PCT Rule 39.1(iv). A search was conducted on the basis of a use of the polymorph or pharmaceutical composition in the preparation of a drug for treating a corresponding disease.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (January 2015)

**INTERNATIONAL SEARCH REPORT**
**Information on patent family members**

International application No.
**PCT/CN2022/092465**

| Patent document cited in search report | Publication date (day/month/year) | Patent family member(s) | Publication date (day/month/year) |
|---|---|---|---|
| WO 2021088839 A1 | 14 May 2021 | AU 2020379709 A1 | 09 June 2022 |
| | | TW 202132313 A | 01 September 2021 |
| | | CA 3156625 A1 | 14 May 2021 |
| CN 102762576 A | 31 October 2012 | US 2016052933 A1 | 25 February 2016 |
| | | US 2014288047 A1 | 25 September 2014 |
| | | SG 10201405049 R A | 30 October 2014 |
| | | HK 1211929 A1 | 03 June 2016 |
| | | EP 2845592 A1 | 11 March 2015 |
| | | RU 2012117398 A | 10 November 2013 |
| | | PT 2483278 E | 05 March 2014 |
| | | WO 2011036280 A1 | 31 March 2011 |
| | | RU 2654068 C1 | 16 May 2018 |
| | | ES 2444779 T3 | 26 February 2014 |
| | | PE 20121025 A1 | 06 August 2012 |
| | | AU 2010299816 A1 | 02 February 2012 |
| | | MX 2012003591 A | 19 April 2012 |
| | | CR 20120121 A | 01 June 2012 |
| | | US 2012244149 A1 | 27 September 2012 |
| | | ES 2570569 T3 | 19 May 2016 |
| | | BR 112012006807 A2 | 03 November 2020 |
| | | PL 2483278 T3 | 30 May 2014 |
| | | DK 2483278 T3 | 13 January 2014 |
| | | HR P20140229 T1 | 11 April 2014 |
| | | EP 2711368 A1 | 26 March 2014 |
| | | JP 2013505917 A | 21 February 2013 |
| | | ZA 201202199 B | 30 January 2013 |
| | | EC SP12011755 A | 30 May 2012 |
| | | IL 217558 D0 | 29 February 2012 |
| | | CO 6491026 A2 | 31 July 2012 |
| | | CA 2772691 A1 | 31 March 2011 |
| | | JP 2014070075 A | 21 April 2014 |
| | | US 2017081341 A1 | 23 March 2017 |
| | | KR 20120065431 A | 20 June 2012 |
| | | TW 201127844 A | 16 August 2011 |
| | | EP 2483278 A1 | 08 August 2012 |
| | | CN 104744491 A | 01 July 2015 |
| | | US 2013079331 A1 | 28 March 2013 |
| | | US 2014058098 A1 | 27 February 2014 |
| | | US 2011076292 A1 | 31 March 2011 |
| | | RS 53164 B | 30 June 2014 |
| | | MA 33531 B1 | 01 August 2012 |
| | | AR 078187 A1 | 19 October 2011 |
| | | NZ 597833 A | 31 January 2014 |
| | | SI 2483278 T1 | 31 March 2014 |
| | | IL 239081 D0 | 30 July 2015 |
| | | MY 160064 A | 15 February 2017 |
| | | CL 2012000754 A1 | 07 September 2012 |
| CN 103562210 A | 05 February 2014 | MX 2013009934 A | 01 October 2013 |
| | | KR 20140032383 A | 14 March 2014 |
| | | BR 112013024122 A2 | 24 September 2019 |

Form PCT/ISA/210 (patent family annex) (January 2015)

**INTERNATIONAL SEARCH REPORT**
**Information on patent family members**

International application No.
**PCT/CN2022/092465**

| Patent document cited in search report | | | Publication date (day/month/year) | Patent family member(s) | | | Publication date (day/month/year) |
|---|---|---|---|---|---|---|---|
| | | | | HK | 1194383 | A1 | 17 October 2014 |
| | | | | US | 2012245144 | A1 | 27 September 2012 |
| | | | | WO | 2012126901 | A1 | 27 September 2012 |
| | | | | JP | 2014509611 | A | 21 April 2014 |
| | | | | RU | 2013143747 | A | 27 April 2015 |
| | | | | CA | 2825966 | A1 | 27 September 2012 |
| | | | | EP | 2688891 | A1 | 29 January 2014 |
| CN | 107995911 | A | 04 May 2018 | JP | 2018519304 | A | 19 July 2018 |
| | | | | HK | 1254517 | A1 | 19 July 2019 |
| | | | | US | 2017002022 | A1 | 05 January 2017 |
| | | | | WO | 2017001658 | A1 | 05 January 2017 |
| | | | | EP | 3317283 | A1 | 09 May 2018 |
| | | | | CN | 111848643 | A | 30 October 2020 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2011036280 A
- WO 2017001658 A
- WO 2017001645 A


### Non-patent literature cited in the description


- **MA K ; CHEUNG SM ; MARSHALL AJ et al.** *Cell Signal,* 2008, vol. 20, 684-694
- **VANHAESEBROECK B ; WATERFIELD MD.** *Exp Cell Res,* 1999, vol. 253, 239-254
- **WU P ; LIU T ; HU Y.** *Curr Med Chem,* 2009, vol. 16, 916-930
- **FALASCA M ; T. MUFFUCCI.** *Biochem Soc Trans,* 2007, vol. 35, 211-214
- **SCHU P ; TAKEGAWA. K ; FRY. M et al.** *Science,* 1993, vol. 260, 88-91
- **KONG D ; YAMORI T.** *Cancer Sci,* 2008, vol. 99, 1734-1740
- **STEELMAN. LS ; CHAPPELL. WH ; ABRAMS. SL et al.** *Aging,* 2011, vol. 3, 192-222
- **DEJAN JURIC et al.** *Cancer Res,* 2012, vol. 72, 1